# FASCICULE DE BREVET EUROPEEN

(11) **EP 0 880 588 B1**
(45) Date de publication et mention de la délivrance du brevet: **27.01.2010**
(21) Numéro de dépôt: 97905212.3
(22) Date de dépôt: 14.02.1997
(51) Int. Cl.: C12N 15/30, C12N 15/86, C07K 14/445, A61K 39/015, C07K 16/20, G01N 33/569, C12N 5/24

(54) **PROTEINE RECOMBINANTE CONTENANT UN FRAGMENT C-TERMINAL DE MSP-1 DE PLASMODIUM**
REKOMBINANTES PROTEIN, DAS EIN C-TERMINALES FRAGMENT VON MSP-1 AUS PLASMODIUM ENTHÄLT
RECOMBINANT PROTEIN CONTAINING A C-TERMINAL FRAGMENT OF PLASMODIUM MSP-1

(30) Priorité: 14.02.1996 FR 9601822
(43) Date de publication de la demande: 02.12.1998
(73) Titulaire: INSTITUT PASTEUR, 75724 Paris Cedex 15 (FR); NEW YORK UNIVERSITY MEDICAL CENTER, New York, NY 10016 (US)
(72) Inventeur: LONGACRE-ANDRE, Shirley, F-75006 Paris (FR); ROTH, Charles, c/o Agnès Rimond, F-92500 Rueil-Malmaison (FR); NATO, Faridabano, F-92160 Antony (FR); BARNWELL, John W., New York, NY 10012 (US); MENDIS, Kamini, Columbo 8 (LK)
(74) Mandataire: Gutmann, Ernest
(86) Numéro de dépôt international: PCT/FR1997/000290
(87) Numéro de publication internationale: WO 1997/030158

(56) Documents cités:
- EP-A- 0 154 454
- WO-A-93/17107
- MOLECULAR AND BIOCHEMICAL PARASITOLOGY, vol. 74, no. 1, 20 Décembre 1995, pages 105-111, XP000603955 LONGACRE, S.: "The Plasmodium cynomolgi merozoite surface protein 1 C-terminal sequence and its homologies with other Plasmodium species" cité dans la demande
- MOLECULAR AND BIOCHEMICAL PARASITOLOGY, vol. 64, no. 2, 1 Janvier 1994, pages 191-205, XP000603954 LONGACRE, S. ET AL.: "Plasmodium vivax merozoite surface protein 1 C-terminal recombinant proteins in baculovirus" cité dans la demande
- MOLECULAR AND BIOCHEMICAL PARASITOLOGY, vol. 60, no. 2, 1 Janvier 1993, pages 303-310, XP000603763 CHAPPEL, J.A. & HOLDER, A.A.: "Monoclonal antibodies that inhibit Plasmodium falciparum invasion in vitro recognise the first growth factor-like domain of merozoite surface protein-1"
- JOURNAL OF IMMUNOLOGY, vol. 149, no. 2, 15 Juillet 1992, BALTIMORE US, pages 548-555, XP000605417 CHANG, S.P. ET AL.: "A carboxy-terminal fragment of Plasmodium falciparum gp195 expressed by a recombinant baculovirus induces antibodies that completely inhibit parasite growth"
- FASEB JOURNAL, vol. 10, no. 6, 2 Juin 1996, page A1117 XP000604872 ANGOV E ET AL: "CHARACTERIZATION OF A RECOMBINANT PLASMODIUM FALCIPARIUM MSP 1 C-TERMINAL FRAGMENT USING CONFORMATION-SPECIFIC MONOCLONAL ANTIBODIES"

## Description

L'invention concerne de nouveaux principes actifs de vaccins dérivés de la protéine majeure de surface de formes mérozoïtes d'un *Plasmodium* infectieux pour des mammifères, notamment l'homme, plus généralement connue sous la désignation MSP-1.

Cette protéine a déjà fait l'objet d'études nombreuses. Elle est synthétisée dans le stade schizonte des parasites du type *Plasmodium*, notamment *Plasmodium falciparum*, et est exprimée sous forme de l'un des constituants majeurs de la surface des mérozoïtes aussi bien pendant le stade hépatique que pendant le stade érythrocytaire du paludisme (1, 2, 3, 4). En raison du caractère prédominant et de la conservation dans toutes les espèces de *Plasmodium* connues de cette protéine, il a été suggéré qu'elle pourrait représenter un candidat pour la constitution de vaccins anti-paludiques (5, 6).

Il en a encore été de même pour des fragments de cette protéine, particulièrement des produits naturels de clivage dont l'on observe la formation, par exemple au cours de l'invasion par le parasite des érythrocytes de l'hôte infecté. Parmi, ces produits de clivage, on relèvera le fragment C-terminal ayant un poids moléculaire de 42 kDa (7,8) qui est à son tour clivé une nouvelle fois en un fragment N-terminal ayant un poids moléculaire apparent conventionnel de 33 kDa et en un fragment C-terminal ayant un poids moléculaire apparent conventionnel de 19 kDa (9) qui reste normalement fixé à la membrane du parasite au terme des modifications dont il est lui-même l'objet, par l'intermédiaire de groupes du type glycosylphosphatidylinositol (GPI) (10, 11).

On le retrouve encore au stade anneau précoce du cycle de développement intraérythrocytaire (15, 16), d'où les observations qui ont été faites que ce fragment de 19 kDa pourrait jouer un rôle non encore connu, mais sans doute essentiel dans les processus réinvasifs. De là découlent les hypothèses déjà formulées dans le passé que cette protéine pourrait constituer une cible particulièrement efficace pour d'éventuels vaccins.

Il sera entendu que les références souvent faites dans ce qui suit à des protéines p42 et p19 issues d'un certain type de *Plasmodium* s'entendent comme se rapportant aux produits de clivage C-terminaux correspondants de la protéine MSP-1 de ce *Plasmodium*, ou, par extension, à des produits contenant sensiblement les mêmes séquences en acides aminés, obtenus par recombinaison génétique ou par synthèse chimique selon les techniques classiques, par exemple par synthétiseur de type « Applied System » ou par synthèse sur phase solide de type « Merrifield ». Pour la commodité du langage, les références à des « p42 recombinantes » et des « p19 recombinantes » se rapportent à des « p42 » et « p19 » obtenues par des techniques comportant au moins une étape de génie génétique.

Devant la difficulté d'obtenir des quantités importantes de parasites pour *P.falciparum* et l'impossibilité de cultiver *P. vivax in vitro*, il est devenu évident que le seul moyen de produire un vaccin antipaludique nécessite un recours aux techniques permettant l'utilisation des peptides ou protéines recombinantes. Mais, le MSP-1 est très difficile à produire en entier à cause de sa grande taille d'environ 200 kDa, un fait qui a conduit les chercheurs à s'intéresser à la partie C-terminale dont la fonction, encore inconnue, est vraisemblablement la plus importante.

Au titre, des protéines recombinantes concernant la partie C-terminale de MSP-1 de *P.falciparum*, qui ont été produites et testées chez le singe (12, 40, 41) on mentionnera :
· une p19 fusionnée avec une glutathione-S-transférase produite dans *E.coli (40),*
· une p42 fusionnée avec une glutathione-S-transférase produite dans *E.coli* (12),
· une p19 fusionnée avec un polypeptide issu d'une anatoxine tétanique et porteur d'épitopes de cellules T auxiliaires produites dans *S.cerevisiae* (12),
· une p42 produite dans un système baculovirus (41).

Une composition contenant la protéine de fusion p19 avec une glutathione-S-transférase produite dans *E.coli* en association avec alun ou liposomes n'a pas exercé d'effet protecteur sur aucun des six singes *Aotus nancymai* vaccinés (40).

Une composition contenant la protéine de fusion p42 avec une glutathione-S-transférase produite dans *E.coli* en association avec un adjuvant complet de Freund n'ont pas exercé d'effet protecteur dans les deux types de singes *Aotus* (*A.nancymai* et *A.vociferans*) auxquels elles avaient été administrées. La protéine p19 produite dans *S.cerevisiae* a exercé un effet protecteur dans deux singes *Aotus* de type *A.nancymai* (12). Par contre elle n'a pas exercé d'effet protecteur dans deux singes *Aotus* de type *A. vociferans*.

Certains chercheurs (Chang et al.) ont également rapporté des essais d'immunisation réalisés chez le lapin avec une protéine recombinante p42 produite dans un système baculovirus et contenant une séquence d'acides aminés en commun avec *P*.*falciparum* (18). Ainsi ces derniers auteurs indiquent-ils que cette p42 recombinante se comporte chez le lapin sensiblement de la même façon que la protéine MSP-1 recombinante entière (gp195). Cette protéine p42 en association avec un adjuvant complet de Freund a fait l'objet d'un essai de vaccination dans un primate non-humain susceptible à l'infection par *P*.*falciparum*., *Aotus, lemurinus grisemembra* (40). Les résultats montrent que 2 sur 3 animaux ont été complètement protégés et le troisième, bien que montrant une parasitémie semblable aux contrôles, avait une période latente plus longue. Il est néanmoins hasardeux de conclure au caractère protecteur chez l'homme des anticorps ainsi induits à l'encontre des parasites eux-mêmes. Rappelons en effet qu'il n'existe pas actuellement de modèles expérimentaux très satisfaisants chez le primate pour *P. vivax* et *P*.*falciparum*. Le modèle *Saimiri*, qui a été développé pour *P*.*falciparum* et *P. vivax*, et le modèle *Aotus* pour *P. falciparum*, sont des systèmes artificiels, nécessitant l'adaptation de souches de parasite et souvent la splénectomie des animaux pour obtenir des parasitémies significatives. En conséquence, les résultats de vaccination provenant de ces modèles ne peuvent avoir qu'une valeur prédictive limitée pour l'Homme.

On peut de toute façon aussi s'interroger sur ce que serait un taux réel de vaccination susceptible d'être éventuellement obtenu avec de telles protéines recombinantes, compte tenu de la constatation -rapportée plus loin- qui a été faite de la présence dans les p42 issues des *Plasmodiums* de la même espèce, et plus particulièrement dans les p33 correspondantes, de régions hypervariables qui rendraient aléatoires en de nombreux cas l'efficacité immunoprotectrice des anticorps induits chez des personnes vaccinées par une p42 issue d'une souche de *Plasmodium* à l'encontre d'une infection par d'autres souches de la même espèce (13).

On peut même supposer que le polymorphisme important de la partie N-terminale du p42 joue un rôle significatif dans l'échappement immunitaire, souvent observé pour ce type de parasites.

La présente invention a pour objectif la production de protéines recombinantes vaccinantes échappant à ces difficultés, dont l'effet protecteur est vérifiable dans des modèles expérimentaux réellement significatifs, ou même directement chez l'homme.

La présente divulgation concerne plus particulièrement des compositions vaccinantes contre un parasite du type *Plasmodium* infectieux pour l'homme, contenant à titre de principe actif une protéine recombinante glycosylée ou non, dont la séquence polypeptidique constitutive essentielle est celle :
- soit d'un fragment C-terminal de 19 kilodaltons (p19) de la protéine 1 de surface (protéine MSP-1) de la forme mérozoïte d'un parasite du type *Plasmodium*, infectieux pour l'Homme, ce fragment C-terminal restant normalement ancré à la surface du parasite au terme de sa phase de pénétration dans des érythrocytes humains, à l'occasion d'un cycle infectieux ;
- soit d'une partie de ce fragment dès lors qu'elle est apte aussi à induire une réponse immune capable d'inhiber une parasitémie in vivo due au parasite correspondant ;
- soit d'un peptide immunologiquement équivalent à ce fragment p19 ou à la susdite partie de ce fragment, et
cette protéine recombinante comportant en outre des épitopes conformationnels instables en milieu réducteur et constituant, de préférence, la majorité des épitopes reconnus par des antisérums humains formés contre le *Plasmodium* correspondant.

La présence de ces épitopes conformationnels pourrait jouer un rôle important dans l'efficacité protectrice du principe actif de vaccins. On les retrouve tout particulièrement dans les principes actifs présentant par ailleurs les autres caractéristiques définies ci-dessus, lorsqu'ils ont été produits dans un système vecteur baculovirus. S'il en est besoin, il est mentionné ci-après que par l'expression « système de vecteur baculovirus » on entend l'ensemble que constitue le vecteur type baculovirus lui-même et les lignées cellulaires, notamment cellules d'insectes transfectables par un baculovirus modifié par une séquence à transférer dans ces lignées cellulaires avec pour résultat l'expression de cette séquence transférée. Des exemples préférés de ces deux partenaires du système baculovirus, ont été décrits dans l'article de Longacre et al. (19). C'est le même système qui a été utilisé dans les exemples qui suivent. Il va naturellement de soi que des variants, tant du baculovirus que des cellules infectables par ce baculovirus peuvent être utilisés en lieu et place de celui qui a été choisi.

En particulier ladite protéine recombinante est reconnue par des antisérums humains formés contre le *Plasmodium* correspondant ou contre un *Plasmodium* homologue lorsqu'elle est à l'état non réduit ou dans un état réduit non irréversible, mais non reconnue ou peu reconnue par ces mêmes antisérums, lorsqu'elle est irréversiblement réduite.

Le caractère instable en milieu réducteur de ces épitopes conformationnels peut être mis en évidence, notamment par le test décrit plus loin dans les exemples, notamment en présence de β-mercaptoéthanol. De même sont indiquées dans des exemples ci-après des conditions expérimentales applicables à l'obtention d'une réduction irréversible des protéines conformes à l'invention.

De ce point de vue, la protéine recombinante produite par S. Longacre et al. (14) peut être mise en oeuvre dans de tels compositions. Il est rappelé que S. Longacre et al. réussirent à produire une p19 recombinante issue de la MSP-1 de *P. vivax* dans un système vecteur à baculovirus contenant une séquence nucléotidique codant pour la p19 de *Plasmodium vivax,* en particulier par transfection de cultures de cellules d'insectes [lignée de *Spodoptera frugiperda* (*Sf9*)] avec des baculovirus vecteurs contenant, sous le contrôle du promoteur de la polyhédrine, une séquence codant pour les fragments peptidiques définis ci-dessous, dont les séquences étaient placées dans l'ordre suivant dans le vecteur baculovirus utilisé :
- fragment 5'-terminal de 35 paires de bases de la séquence signal de la polyhédrine, dont avait été muté (en ATT) le codon méthionine d'initiation de l'expression de cette protéine;
- un fragment 5'-terminal nucléotidique codant pour un peptide de 32 acides aminés correspondant à la partie N-terminale de MSP-1, y compris le peptide signal de MSP-1 ;
- soit une séquence nucléotidique codant pour la p19, soit une séquence codant pour la p42 de la protéine MSP-1 de *Plasmodium vivax*, ces séquences étant aussi, selon le cas, soit pourvues (formes « ancrées »), soit dépourvues (formes solubles) des régions d'extrémité 3' de ces séquences de nucléotides dont les produits d'expression C-terminaux extrêmes sont réputés jouer un rôle essentiel dans l'ancrage de la protéine finale p19 sur la membrane du parasite ;
- 2 codons stop TAA.

Pour la p42, les séquences dérivées de la région C-terminale de MSP-1 s'étendaient par conséquent de l'acide aminé Asp 1325 à l'acide aminé Leu 1726 (forme ancrée) ou à l'acide aminé Ser 1705 (forme soluble) et pour la p19, les séquences s'étendaient de l'acide aminé Ile 1602 à l'acide aminé Leu 1726 (forme ancrée) ou à l'acide aminé Ser 1705 (forme soluble), étant entendu que les séquences en acides aminés complètes de p42 et p19 dont les acides aminés initiaux et terminaux ont été indiqués dans ce qui précède découlent du gène de l'isolat Belem de *P. vivax* qui a été séquencé (20).

Des résultats semblables ont été obtenus en mettant en oeuvre dans les mêmes systèmes de vecteurs des séquences nucléotidiques codant pour la p42 et la p19 de *Plasmodium cynomolgi. P.cynomolgi* présente un double intérêt : c'est une espèce parasitaire très proche de *P.vivax* qui est infectieuse pour le macaque. Il peut aussi infecter l'Homme. On a également accès à des hôtes naturels de *P.cynomolgi*, les singes rhésus et les singes toques, pour tester l'efficacité de protection du MSP-1 de *P.cynomolgi* dans des systèmes naturels. Le singe rhésus est considéré comme une des espèces les plus représentatives des réactions immunitaires chez l'Homme.

En particulier, on a obtenu d'excellents résultats dans des essais de vaccination réalisés chez le singe toque avec deux polypeptides recombinants : la p42 et, surtout, la p19 solubles, dérivées de *P.cynomolgi*, respectivement produites dans un système baculovirus et purifiées sur colonne d'affinité avec des anticorps monoclonaux reconnaissant les régions correspondantes de la protéine MSP-1 native. Les observations suivantes ont été faites : les six singes immunisés avec le seul p19 (trois singes) et le 19 et p42 ensemble (3 singes) ont tous témoigné d'une immunité presque stérile après infection d'épreuve. Les résultats obtenus chez les trois singes immunisés avec le p42 ont été moins significatifs. Deux d'entre eux se sont comportés comme les précédents, mais si le troisième a manifesté une parasitémie moins importante que des témoins immunisés avec un tampon PBS en présence de l'adjuvant de Freund (3 singes) ou non immunisés (3 singes), elle n'en n'était pas moins patente.

Une deuxième infection d'épreuve montrant que les singes ayant reçu la p19 seule étaient protégés au moins pendant six mois. Un deuxième essai de vaccination avec la p19 en association avec alun dans ce système (*P.cynomolgi*/singe toque) a démontré une protection significative pour 2 des 3 singes. C'est la première fois que la MSP-1 ou un autre antigène recombinant a démontré un effet protecteur en présence d'alun (42).

Les résultats des essais particulièrement efficaces réalisés chez le macaque avec des polypeptides recombinants produits dans un système baculovirus mettant en oeuvre une p19 recombinante de *P.cynomolgi*, établissent que des polypeptides recombinants contenant respectivement des p19 recombinantes issus d'autres *Plasmodiums* doivent se comporter de la même manière. Ils sont « plus parlants » pour le paludisme chez l'Homme que les résultats d'essais réalisés avec *P.vivax* ou *P.falciparum* dans leurs « hôtes artificiels ».

Les protéines recombinantes de baculovirus, dérivées d'une partie C-terminale de MSP-1 (p19), ont un effet protecteur antipaludique très significatif dans un système naturel, qui constitue le modèle d'évaluation de l'effet protecteur de MSP-1 le plus représentatif pour l'homme.

L'effet protecteur obtenu pourrait être d'autant meilleur que la forme p19 est dépourvue de la région hypervariable de la partie N-terminale du p42, dont l'effet peut être délétère dans des situations naturelles dans lesquelles le sujet vacciné est confronté à un polymorphisme important. Aussi la p19 semble posséder des épitopes spécifiques qui ne sont pas présents dans la p42.

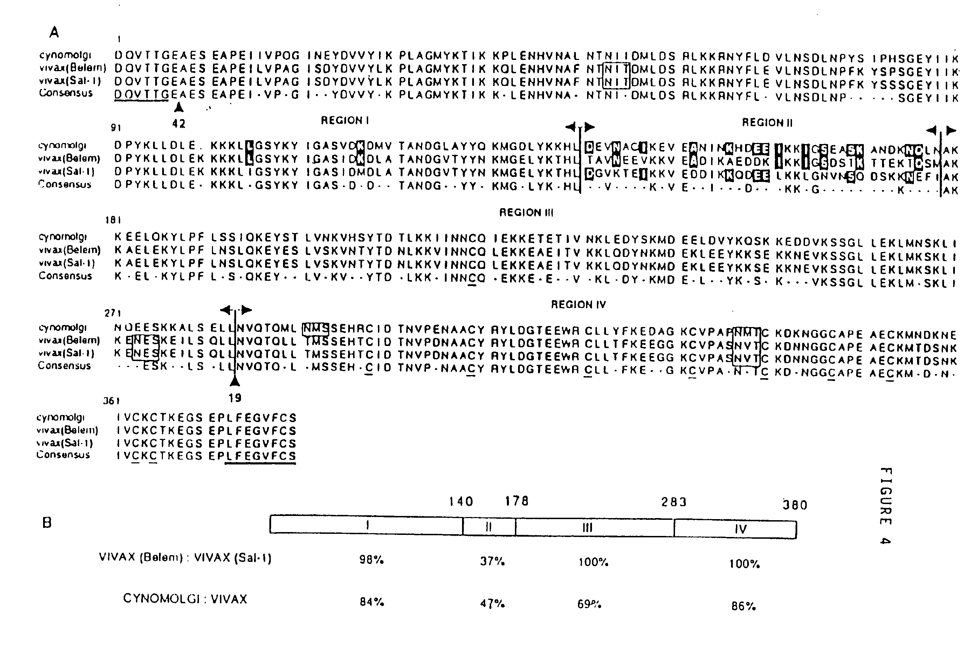

Le fragment C-terminal de 19 kDa dont la séquence est présente dans le principe actif du vaccin peut être limité à la séquence de la p19 elle-même, en l'absence de toute séquence polypeptidique normalement en amont de la séquence de p19 dans la protéine MSP-1 correspondante. Il va néanmoins de soi que la séquence polypeptidique constitutive essentielle du principe actif peut encore comporter une séquence polypeptidique du côté C-terminal appartenant au fragment N-terminal de 33 kDa (p33) encore associée à la p19 dans la p42 correspondante, avant le clivage naturel de cette dernière, et cela chaque fois que la présence de ce fragment n'est pas de nature à modifier les propriétés immunologiques du principe actif du vaccin. Comme cela sera vu plus loin, notamment à propos de la description des exemples, les séquences C-terminales de la p33 de souches diverses d'une même espèce de *Plasmodium* (voir la partie C-terminale des séquences peptidiques de la « région III » de la figure 4), présentent aussi encore un degré d'homologie ou de conservation substantielle de la séquence, par exemple de l'ordre de 80% au moins, dans différentes variétés de *Plasmodiums* infectieux pour l'homme, de sorte qu'elles ne sont pas de nature à modifier fondamentalement les propriétés vaccinantes du principe actif (dont la séquence correspond à la région IV) de la figure 4, en particulier dans l'hypothèse qui découle de cette figure le site de clivage présumé entre la p19 et la région III de la p33 se situe entre les résidus leucine et asparagine dans une région particulièrement bien conservée (LNVQTQ).

Normalement la séquence polypeptidique C-terminale de la p33, lorsqu'elle est présente, comprend moins de 50 résidus d'acides aminés, voire même moins de 35, ou même moins de 10 résidus d'acides aminés.

A l'inverse, la séquence polypeptidique constitutive essentielle du principe actif de vaccin peut ne pas comporter la totalité de la séquence codant pour la p19, naturellement sous réserve que cette dernière conserve la capacité d'induire des anticorps protecteurs contre le parasite. En particulier la susdite « partie de fragment a un poids moléculaire de 10 à 25 kDa, notamment de 10 à 15 kDa. De préférence, cette partie de fragment polypeptidique contient au moins l'une des deux régions EGF (abréviation de l'expression anglaise « Epidermal Growth Factor »).

Il est clair que l'homme du métier est à même de faire la différence entre les fragments actifs et ceux qui cesseraient de l'être, notamment de façon expérimentale en produisant des vecteurs modifiés contenant des insérats issus de la p19 de longueurs différentes, respectivement isolés à partir des fragments obtenus à partir de la séquence codante pour la p19, par réaction avec des enzymes de restriction appropriés, ou encore par des enzymes exonucléolytiques qui auraient été maintenus au contact du fragment codant pour la p19 pendant des temps variables ; la capacité des produits d'expression de ces insérats dans des cellules eucaryotes correspondantes, notamment des cellules d'insectes, transformées par les vecteurs modifiés correspondants, à exercer un effet protecteur, pouvant alors être testée, notamment dans les conditions expérimentales qui seront décrites plus loin, à propos des exemples. En particulier, les produits d'expression de ces insérats doivent être aptes à inhiber une parasitémie induite *in vivo* par le parasite correspondant entier.

De même, la présente divulgation inclut toutes compositions vaccinantes dans lesquelles la séquence polypeptidique constitutive essentielle du principe actif serait constitué d'un peptide capable d'induire une réponse immunologique de type cellulaire et/ou humorale équivalente à celle produite par la p19 ou au fragment tel qu'il vient d'être défini, dès lors que l'addition, la délétion ou la substitution dans sa séquence de certains acides aminés par d'autres n'entraîneraient pas une modification importante de la capacité du peptide ainsi modifié -ci-après appelé « peptide immunologiquement équivalent »- à aussi inhiber la susdite parasitémie.

Le fragment de la p19 peut naturellement aussi être associé que ce soit du côté N-terminal ou du côté C-Terminal ou par l'intermédiaire d'une liaison peptidique à un autre fragment de protéine plasmodiale ayant un potentiel vaccinant comme par exemple une protéine (« Duffy Binding Protein » de P. vivax (29) ou EBA-175 de P. falciparum (30) et (31) dont une région est spécifiquement riche en cystéine), sous réserve que ne soit pas altérée, au contraire amplifiée, sa capacité d'inhiber une parasitémie normalement introduite in vivo par le parasite correspondant.

Le fragment codant pour la p19 ou une partie de celle-ci, peut contenir également, en amont de l'extrémité N-terminale de p19, une séquence peptidique encore différente, par exemple à un fragment C-terminal du peptide signal utilisé, tel que celui de la protéine MSP-1. Cette séquence comprend de préférence moins de 50 acides aminés, par exemple de 10 à 40 acides aminés.

Ces observations s'étendent de la même façon à des p19 issues d'autres *Plasmodium,* en particulier *P.falciparum*, l'espèce dominante des parasites, responsable d'une des formes les plus graves de paludisme.

Mais les techniques rappelées ci-dessus pour la production dans un système de baculovirus d'une p19 recombinante issue de *P. vivax* ou *P.cynomolgi* sont difficilement transposables telles quelles à la production d'une p19 recombinante de *P.falciparum* avec un rendement satisfaisant, ne fût-ce que pour obtenir des quantités appréciables autorisant la réalisation d'essais d'immunoprotection.

La présente divulgation fournit également un procédé remédiant en grande partie à cette difficulté. Il devient aussi possible d'obtenir des rendements beaucoup plus importants en p19 de *P.falciparum* -et d'autres *Plasmodiums* lorsque l'on rencontre des difficultés semblables- en mettant en oeuvre une séquence nucléotidique synthétique de substitution à la séquence nucléotidique naturelle codant pour la p19 de *Plasmodium falciparum* dans un vecteur d'expression d'un système baculovirus, cette séquence nucléotidique synthétique codant pour la même p19, mais étant caractérisée par une proportion de nucléotides G et C plus élevée que dans la séquence nucléotidique naturelle.

En d'autres termes, l'invention découle de la découverte que l'expression dans un système baculovirus d'une séquence nucléotidique codant pour une p19, était apparemment liée à une compatibilité améliorée des codons successifs de la séquence nucléotidique à exprimer avec la « machinerie cellulaire » des cellules hôtes transformables par des baculovirus, à l'instar de ce qui est observé pour les séquences nucléotidiques naturelles normalement contenues dans ces baculovirus et exprimées dans les cellules hôtes infectées ; d'où la mauvaise expression, sinon parfois l'absence totale d'expression d'une séquence nucléotidique native de *P.falciparum ;* d'où également une explication possible à l'expression plus efficace observée de la p19 de *P. vivax* dans un système baculovirus par Longacre et al. (14) et, comme les inventeurs l'ont aussi constaté, de la séquence de *P.cynomolgi* à partir des séquences nucléotidiques p19 natives correspondantes, en raison de leurs teneurs relatives en nucléotides G et C beaucoup plus élevées que celles des séquences nucléotidiques natives codant pour les p19 de *P.falciparum*.

La présente divulgation concerne donc aussi, plus généralement, un vecteur modifié du type baculovirus recombinant contenant sous le contrôle d'un promoteur contenu dans ce vecteur et susceptible d'être reconnu par des cellules transfectables par ce vecteur, une première séquence nucléotidique codant pour un peptide signal exploitable par un système baculovirus, caractérisé par une deuxième séquence nucléotidique en aval de la première, également sous le contrôle de ce promoteur et code pour la séquence peptidique :
- soit d'un fragment peptidique C-terminal de 19 kilodaltons (p19) de la protéine 1 de surface (protéine MSP-1) de la forme mérozoïte d'un parasite du type *Plasmodium* autre que *Plasmodium vivax* et infectieux pour l'Homme, ce fragment C-terminal restant normalement ancré à la surface du parasite au terme de sa pénétration dans des érythrocytes humains, à l'occasion d'un cycle infectieux ;
- soit d'une partie de ce fragment peptidique dès lors que le produit d'expression de la deuxième séquence dans un système baculovirus est apte aussi à induire une réponse immune capable d'inhiber une parasitémie in vivo due au parasite correspondant ;
- soit d'un peptide immunologiquement équivalent dérivé du susdit fragment peptidique C-terminal (p19) ou de la susdite partie de fragment peptidique par addition, délétion ou substitution d'acides aminés n'entraînant pas une modification importante de la capacité de ce peptide immunologiquement équivalent à induire une réponse immunologique de type cellulaire et/ou humorale semblable à celle produite par ce fragment peptidique p19 ou à la susdite partie de ce fragment, et
ladite séquence nucléotidique ayant le cas échéant une teneur en nucléotides G et C comprise entre 40 et 60%, de préférence d'au moins 50% de la totalité des nucléotides dont elle est constituée. Cette séquence peut être obtenue par construction d'un gène synthétique dans lequel les codons naturels ont été changés par des codons riches en G/C sans que leur traduction soit modifiée (maintien de la séquence peptidique).

En l'occurrence ladite séquence nucléotidique, fournie par un ADN synthétique, peut présenter au moins 10% de codons modifiés par rapport à la séquence du gène ou du cDNA naturel tout en conservant les caractéristiques de la séquence naturelle traduite, c'est-à-dire le maintien de la séquence en amino-acides.

Cela étant, on n'exclut pas que cette teneur en nucléotides G et C pourrait être davantage accrue, dès lors que les modifications qui en résulteraient quant à la séquence en acides aminés du peptide recombinant -ou peptide immunologiquement équivalent-produit n'entraîneraient pas une perte des propriétés immunologiques, voire protectrices, des protéines recombinantes formées, notamment dans les essais qui seront illustrés plus loin.

Ces observations s'appliquent naturellement à d'autres *Plasmodium* infectieux pour l'homme, en particulier dès lors que des séquences nucléotidiques natives codant pour les p19 correspondantes, auraient des teneurs en nucléotides T et A difficilement compatibles avec une expression efficace dans un système baculovirus.

La séquence codant pour le signal utilisé peut être celle normalement associée à la séquence native du *Plasmodium* concerné. Mais elle peut également être issue d'un autre *Plasmodium*, par exemple *P*.*vivax* ou *P*.*cynomolgi* ou d'un autre organisme s'il est susceptible d'être reconnu en tant que signal dans un système baculovirus.

La séquence codant pour la p19 ou un fragment de celle-ci au sein du vecteur considéré est, le cas échéant, dépourvue de la séquence d'ancrage de la protéine native au parasite dont elle est issue, cas dans lequel la protéine exprimée est en général excrétée dans le milieu de culture (forme soluble). Il est d'ailleurs remarquable à cet égard que dans les conditions de la présente divulgation, les formes solubles et ancrées de protéines recombinantes produites, en particulier lorsqu'elles sont issues de *P*.*falciparum* ou *P*.*cynomolgi* ou *P. vivax*, ont tendance à former des oligomères, cette propriété pouvant être à l'origine des immunogénicités accrues des protéines recombinantes formées.

La présente divulgation concerne tout autant les vecteurs dans lesquels la séquence codante contient la séquence d'extrémité 3' terminale codant pour la séquence d'extrémité C'-terminale hydrophobe de la p19 et qui est normalement impliquée dans l'induction de l'ancrage de la protéine native à la membrane cellulaire de l'hôte dans lequel elle est exprimée. Cette région d'extrémité 3'-terminale peut d'ailleurs être hétérologue vis-à-vis de la séquence codant pour la partie soluble de p19, par exemple correspondre à la séquence 3'-terminale issue de *P. vivax* ou d'un autre organisme dès lors qu'elle code pour une séquence d'ancrage de l'ensemble de la protéine recombinante produite à la membrane de l'hôte cellulaire du système baculovirus mis en oeuvre. A titre d'exemple de telles séquences d'ancrage on cite la GPI de l'antigène CD59 exprimable dans des cellules d'insectes du type *Spodoptera frugiperda* (32) ou la GPI d'une protéine humaine CD14 (33).

La présente divulgation concerne naturellement aussi les protéines recombinantes, ces protéines comportant des épitopes conformationnels reconnus par des sérums humains formés contre le *Plasmodium* correspondant.

D'une façon générale, la présente divulgation concerne également toute protéine recombinante du type indiqué ci-dessus, dès lors qu'elle comporte des épitopes conformationnels tels que produits dans le système baculovirus, notamment ceux qui se trouvent être instables en milieu réducteur.

La présente divulgation concerne naturellement lesdites protéines recombinantes, qu'elles soient sous la forme dite soluble ou sous la forme pourvue d'une région d'ancrage, en particulier aux hôtes cellulaires mis en oeuvre dans le système baculovirus.

Entrent également dans le champ de la présente divulgation, les oligomères produits spontanément dans les systèmes baculovirus utilisés ou produits a posteriori, en ayant recours à des techniques classiques d'oligomérisation de protéines. La technique couramment utilisée met en oeuvre le glutaraldéhyde. Mais tout système classique de pontage entre des fonctions respectivement amine et carboxyle, telles qu'on les retrouvent dans les protéines pourra également être utilisé. A titre d'exemples, on peut mettre en oeuvre l'une quelconque des techniques décrites dans la demande de brevet européen 0602079.

Par « oligomère » on entend une molécule contenant de 2 à 50 unités monomère, chacune de ces unités monomère contenant la p19 ou un fragment de celle-ci, tel que défini ci-dessus, capable de former un agrégat. La présente divulgation porte également sur tout produit de conjugaison entre une p19 ou fragment de p19 tel que défini ci-dessus, d'une part, et une molécule porteuse -par exemple une polylysine-alanine- utilisable pour la production des vaccins, d'autre part, par l'intermédiaire de liaisons covalentes ou non. Les compositions vaccinantes les mettant en oeuvre font également partie de la présente divulgation.

La présente divulgation concerne également les compositions de vaccins mettant en oeuvre ces protéines recombinantes oligomères ou conjugués, y inclus d'ailleurs les protéines issues de *Plasmodium vivax*, ces observations s'étendant également aux oligomères de ces protéines recombinantes.

Font également partie de la présente divulgation les compositions dans lesquelles les protéines recombinantes susmentionnées sont associées à un adjuvant, par exemple un alun. Les protéines recombinantes comportant l'extrême région C-terminale permettant leur ancrage à la membrane des cellules dans lesquelles elles sont produites sont avantageusement utilisées en combinaison avec des lipides aptes à former des liposomes et appropriés à la production de vaccins. Sans qu'il y ait eu lieu de s'y limiter, on peut avoir recours aux lipides décrits à cet effet par exemple dans l'ouvrage intitulé « Les liposomes aspects technologique, biologique et pharmacologique » de J. Delattre et al., édition INSERM, 1993.

La présence de la région d'ancrage dans la protéine recombinante, qu'il s'agisse d'une région d'ancrage homologue ou hétérologue vis-à-vis de la partie vaccinante proprement dite, est de nature à favoriser la production d'anticorps cytophiles, notamment du type IgG₂ₐ et IgG_{2b} chez la souris qui pourraient avoir une activité protectrice particulièrement élevée, au point que l'on pourrait se dispenser d'associer les principes actifs de vaccins ainsi constitués avec des adjuvants autres que les lipides utilisés pour la constitution des formes liposomiques. Il s'agirait là d'un avantage important, puisque les liposomes peuvent être lyophilisés dans des conditions permettant leur stockage et leur transport, sans que des chaînes de froid ne soient alors indispensables.

D'autres caractéristiques de l'invention apparaîtront encore au cours de la description qui suit d'exemples de protéines recombinantes et des conditions dans lesquelles elles peuvent être produites, sans que ces exemples soient de nature à limiter la portée de l'invention.

### Description de la construction PfMSP1ₚ₁₉S (soluble) (p19 soluble issue de P.falciparum)

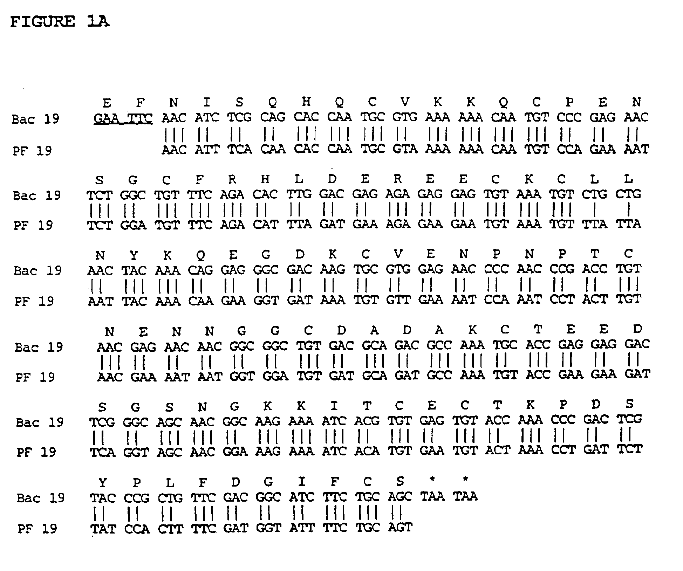

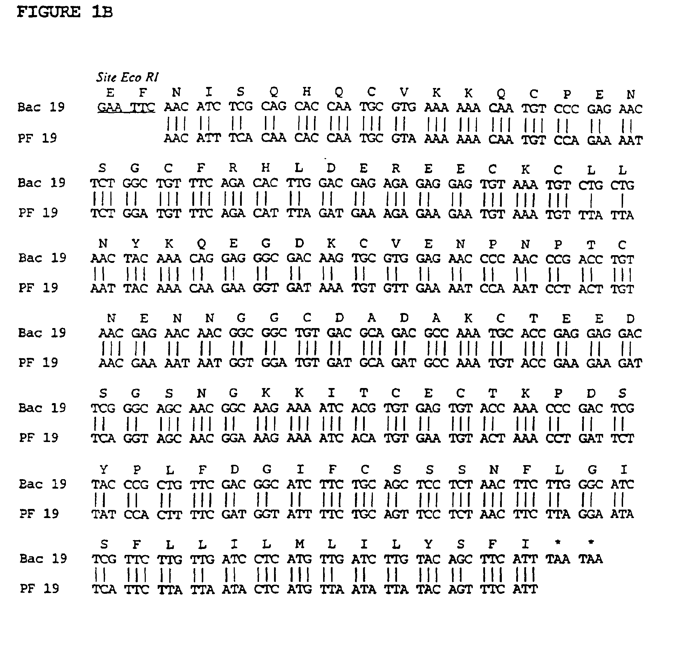

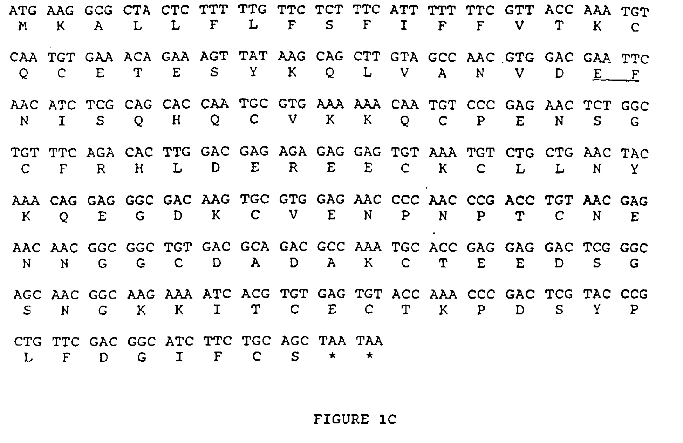

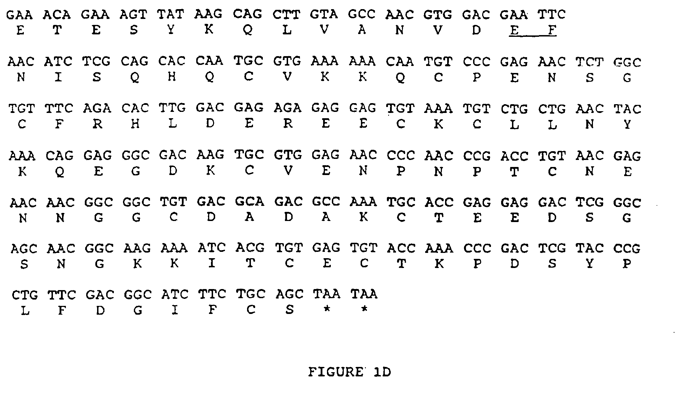

La construction recombinante PfMSP1ₚ₁₉S contient de l'ADN correspondant aux 8 paires de base de la séquence leader et les 32 premiers acides aminés de MSP1 de *Plasmodium vivax* de Met₁ à Asp₃₂ (isolat Belem ; Del Portillo et al. 1991. P.N.A.S. 88, 4030.) suivis par un GluPhe, dû au site EcoR1 faisant la liaison des deux fragments. Le tout est suivi par le gène synthétique, décrit dans Figure 1, codant le *Plasmodium falciparum* MSP1ₚ₁₉ de Asn₁₆₁₃ à Ser₁₇₀₅ (isolat Uganda-Palo Alto ; Chang et al. 1988. Exp. Parasitol. 67,1). La construction est terminée par deux codons stop de TAA. Cette construction donne lieu à une protéine recombinante qui est sécrétée dans le surnageant de culture des cellules infectées.

De la même façon et à des fins de comparaisons, on a produit une construction recombinante dans des conditions semblables à celles utilisées pour la production de la p19 ci-dessus, mais en travaillant avec une séquence codante consistant en une copie directe de l'ADN correspondant de la souche *P.falciparum* (FUP) décrite par Chang et al., Exp. Parasit. 67,1; 1989. La copie de ce gène naturel (s'étendant de l'asparagine 1613 à la serine 1705) a été formée par PCR à partir du gène natif.

On a représenté dans la **Figure 1A** les séquences à la fois du gène synthétique (Bac19) et du « gène natif » (PF19).

On remarque que 57 codons sur les 93 codons de la séquence codante native pour la p19 issue de *P.falciparum* ont été modifiés (pour ce qui est du troisième nucléotide dans 55 d'entre eux et du premier et troisième nucléotides dans les deux codons restant). Des codons nouveaux ont été ajoutés à l'extrémité 5' pour introduire le peptide signal dans les conditions qui ont été indiquées ci-dessus et pour introduire un site EcoRI pour le clonage, d'une part, et de même ont été ajoutés deux codons stop non présents dans la p19 de *P.falciparum* aux fins d'obtenir des signaux de terminaison de l'expression. Les lettres individualisées placées respectivement au dessus des codons successifs correspondent aux acides aminés respectifs successifs. Les astérisques (*) se rapportent aux codons stop. Les lignes verticales soulignent les nucléotides qui sont les mêmes dans les deux séquences.

### Description de la construction PfMSP1ₚ₁₉A (ancré GPI) (p19 ancrée de P.falciparum)

La construction PfMSP1ₚ₁₉A a des caractéristiques de la précédente sauf que la séquence synthétique (**Figure 1B**) code pour le MSP1ₚ₁₉ de *Plasmodium falciparum* (isolat Uganda-Palo alto) de Asn₁₆₁₃ à Ile₁₇₂₆, suivie par deux codons stop de TAA. Cette construction donne lieu à une protéine recombinante qui est ancrée dans la membrane plasmique des cellules infectées par une structure du type glycosyl phosphatidyl inositol (GPI).

La **figure 1C** est représentative de la séquence de la protéine recombinante PfMSP1_{P19}S avant coupure de la séquence signal.

La **figure 1D** est représentative de la séquence de la protéine recombinante PfMSP1ₚ₁₉S après coupure de la séquence signal.

Les acides aminés soulignés dans les figures 1C et 1D proviennent du site EcoR1 utilisé pour joindre les séquences nucléotidiques dérivées de la partie N-terminale de MSP1 de *P.vivax* (avec séquence signal) et de MSP1ₚ₁₉ de *P.falciparum*.

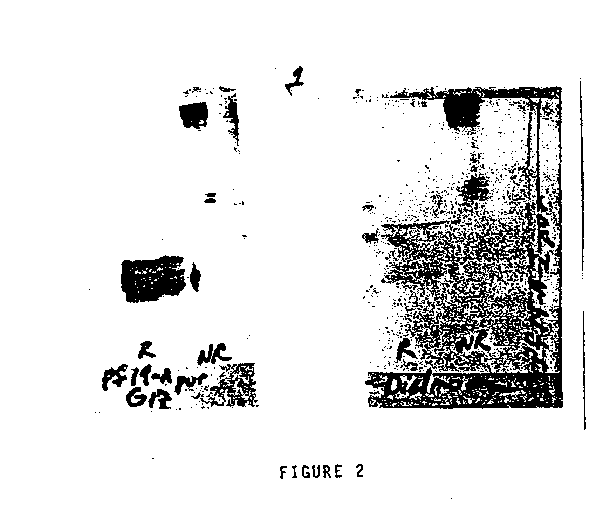

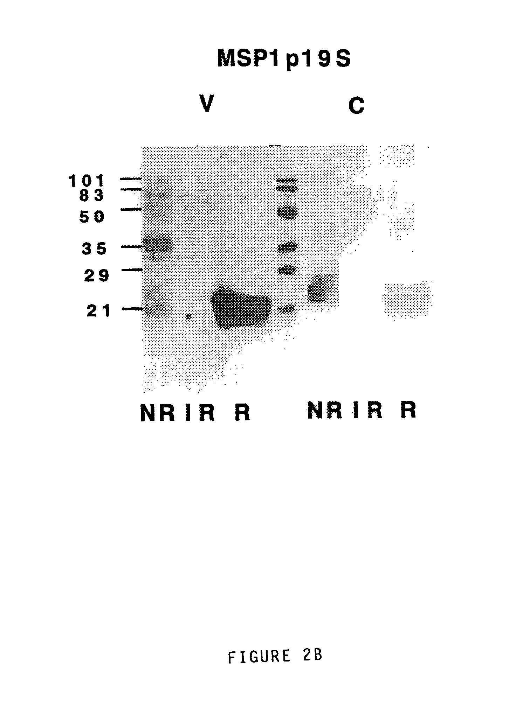

**Figure 2** - L'antigène recombinant PfMSP1ₚ₁₉ soluble purifié par immunoaffinité a été analysé par immunoblot après SDS-PAGE en présence (réduit) ou absence (non réduit) de B-mercaptoéthanol. Les échantillons sont chargées sur gel après chauffage à 95°C en présence de 2% SDS. Dans ces conditions seulement des liaisons du type covalent (ponts disulfure) peuvent résister à la désagrégation. Le blot de gauche a été révélé avec un anticorps monoclonal qui réagit avec un épitope linéaire de la p19 naturelle. Le blot de droite a été révélé avec un mélange de 13 antisera humains provenant des sujets avec une immunité acquise au paludisme dû à *Plasmodium falciparum*. Ces résultats montrent que la molécule recombinante de baculovirus reproduit bien les épitopes conformationnels en forme de polymère qui sont reconnus en majorité par l'antiserum humain.

**Figure 2B** Analyse par immunoblot avec antisérum humain du MSP-1 p19 purifié de *P. vivax* et *P. cynomolgi* recombinant dans des conditions non-réduites (NR), réduite seulement dans le milieu de chargement (R) et réduite irréversiblement (IR):

Ce travail est basé sur l'idée que le système d'expression baculovirus reproduit correctement et en grande proportion les épitopes conformationnels présents *in vivo* sur la partie C-terminale de MSP-1. Le meilleur moyen de mesurer cette propriété (et peut être le seul moyen possible en absence des protéines natives purifiées correspondant au p19) est d'étudier la réactivité des protéines recombinantes avec l'antisérum des individus exposés au paludisme, ceci étant un reflet des protéines natives telles qu'elles sont "vues" par le système immunitaire humain.

Donc, les antigènes recombinants PvMSP-1 p19 et Pc MSP-1 p19 solubles purifié par immunoaffinité ont été analysé par immunoblot après SDS-PAGE (15%) en présence (réduit) ou absence (non-réduit) de DTT Les échantillons sont chargées sur gel après chauffage à 95°C en présence de 2% SDS. La réduction irréversible était faite comme suit: la protéine est resuspendue en 0.2 M Tris-HCl, pH 8.4, 100 mM DTT, 1.0% SDS et chauffée 30 minutes à 70°C. Après dilution avec l'eau, de l'acrylamide est ajouté à une concentration finale de 2 M et la mixture est incubée sous azote sans lumière 1 heure à 37°C. L'immunoblot a été révélé avec un mélange de 25 antisera humains provenant des sujets avec une immunité acquise au paludisme dû à *Plasmodium vivax*. V et C désigne respectivement les protéines dérivées de MSP-1 de *P. vivax* et P. *cynomolgi*. Il est remarquable que les protéines recombinantes réduites de façon irréversible ne montrent aucune réactivité avec l'antisérum humain tandis que les protéines réduites de façon non-irréversible ou non-réduites montrent une bonne réactivité. (Le Pv MSP-1 p19 non-réduit est un peu faible parce que dans son état glycosylé il ne se lie pas très bien au papier nitrocellulose.) Ces résultats montrent que la reconnaissance des molécules MSP-1 p19 de baculovirus par l'antisérum humain est en grande partie, sinon complètement dépendant des épitopes conformationnels sensibles à la réduction qui sont reproduits dans ce système.

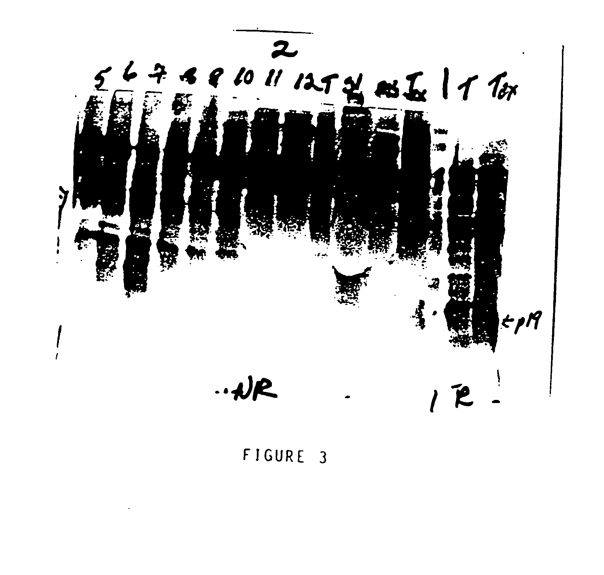

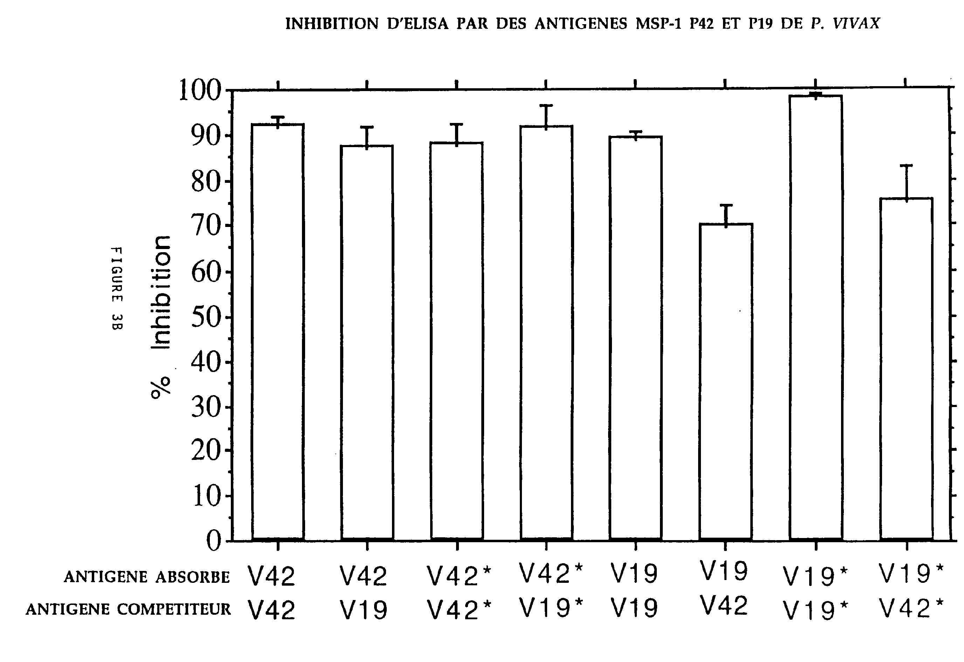

**Figure 3** - L'antigène recombinant PvMSP1_{P42} soluble (Longacre et al. 1994, op.cit.) a été incubé pendant 5 heures à 37° en présence des fractions de protéines dérivées des mérozoïtes de *P*.*falciparum* et séparées par isoélectrofocalisation. Par la suite les échantillons ont été analysés par immunoblot en présence (réduit) ou absence (non réduit) de B-mercaptoéthanol. Les fractions 5 à 12 d'isoélectrofocalisation, ainsi que deux extraits totaux de mérozoïtes faits en présence (Tex) ou absence (T) de détergent ont été analysés. L'immunoblot a été révélé avec des anticorps monoclonaux spécifiques pour le MSP1ₚ₄₂ et ₚ₁₉ de *P.vivax*. Les résultats suggèrent qu'il y a une activité protéolytique dans les mérozoïtes de *P*.*falciparum* qui peut être extraite en détergent. La digestion du p42 dans certaines fractions semble provoquer une polymérisation des produits de digestion (p19) ; cette polymérisation est probablement liée à la formation de ponts disulfure puisqu'en présence de B-mercaptoéthanol, les formes de haut poids moléculaire disparaissent en faveur d'une molécule d'environ 19 kDa (Tex-R). La polymérisation du p19 observée dans ces expériences pourrait donc être une propriété intrinsèque de cette molécule *in vivo*.

**Figure 3B****:** La contribution différentielle des antigènes p42 et p19 à la réponse humaine anti-MSP-1 de *P. vivax*.

La reconnaissance des antigènes MSP-1 p42 et p19 de *P. vivax* par l'antisérum des individus ayant une immunité acquise à *P. vivax* a été comparée par la technique d'inhibition d'ELISA comme suit: un mélange de 25 antisérums humains provenant des sujets avec une immunité acquise au paludisme dû à *P. vivax* a été dilué au 1:5000 et incubé 4 heures à température ambiante soit seul, soit en présence d'une solution de 1 mM p42 ou p19 recombinant purifié de *P. vivax*. Cette mixture a été transférée dans un puits de microtitre auparavant revêtu pendant 18 heures à 4°C par 500 ng ml⁻¹ p42 ou p19 recombinant purifié absorbé, et incubée 30 minutes à température ambiante. Après lavage avec PBS contenant 0.1% Tween 20, un anti-souris IgG de chèvre conjugué avec peroxydase a été ajouté et la mixture a été incubée 1 heure à 37°C et l'activité enzymatique a été révélée par lecture de la densité optique à 492 nm. La pourcentage d'inhibition a été calculée basé sur des valeurs de 100% de réactivité d'antisérum avec l'antigène revêtu en plaque de microtitre en absence d'un antigène compétiteur. Les données statistiques ont été calculées en utilisant le programme Statview. Chaque barre représente l'inhibition moyenne en pourcentage d'une paire d'antigènes compétiteur / absorbé basée sur 4 à 12 déterminations et les lignes verticales correspondent à un intervalle de 95% de confiance. Les étoiles (*) désignent des antigènes produits en présence de tunicamycine, donc sans N-glycosylation. Les paramètres importants de ces mesures sont la dilution d'antisérum à 1:5000 qui est dans la région sensible des courbes d'ELISA et les concentrations d'antigènes compétiteurs à 1 mM qui incluent la compétition par les épitopes de basse affinité. Donc, les données reflètent la similitude maximum entre les deux antigènes comparés. Les résultats montrent que la plupart, si ce n'est pas tous les épitopes du p42 reconnus par l'antisérum humain sont présents sur le p19 puisqu'en présence de ce dernier, la réactivité de l'antisérum humain contre le p42 est inhibée autant que par l'antigène p42 lui même. Mais, au contraire, environ 20% des épitopes du p19 reconnus par l'antisérum humain ne le sont pas ou ne sont pas accessibles sur le p42, puisque la réactivité de l'antisérum humain contre le p19 est bien moins inhibée par le p42 que par le p19 lui même. Des tels épitopes spécifiques du p19 peuvent être constitué ou révélés seulement après la coupure du p42 en p19 et p33. Ces résultats ne sont pas affectés par la glycosylation montrant que l'effet est bien dû à une différence entre les composants peptidiques du p19 et p42 et pas à une différence de glycosylation. Ces résultats souligne le fait que le p19 a une identité immunologique distincte du p42.

### Description de la construction PcMSP1ₚ₁₉S (soluble) (p19 soluble de P.cynomolgi)

L'ADN utilisé pour la construction susdite a été obtenu à partir d'un clone de la souche de *Plasmodium cynomolgi ceylonesis* (22-23). Cette souche a été maintenue par des passages successifs dans son hôte naturel (*Macaca sinica*) et des transmissions cycliques par l'intermédiaire de moustiques (27).

Des parasites sanguins ont été obtenus à partir des singes infectés au stade schizonte mature quand les parasitémies ont atteint un niveau de 5%. Ils ont alors été purifiés selon la méthodes décrites dans (25). L'ADN a ensuite été extrait comme décrit dans (26).

Un fragment de 1200 paires de base a ensuite été produit en ayant recours à la réaction PCR mettant en oeuvre les oligonucléotides soulignés dans la **Figure 4** et issus de *P.vivax*. L'oligonucléotide 5' comprenait un site de restriction EcoRI et l'oligonucléotide 3' deux codons synthétiques stop TAA suivis d'un site de restriction BgIII. Ce fragment a été introduit par ligation et par l'intermédiaire de ces sites EcoRI et BgIII dans le plasmide pVLSV₂₀₀ contenant déjà la séquence signal de la protéine MSP-1 de *P*.*vivax* (19). Le nouveau plasmide (pVLSV₂₀₀C₄₂) a été utilisé pour l'analyse de séquences d'ADN.

Les séquences de *P*.*cynomolgi* et des séquences correspondantes de *P*.*vivax* ont été mises en alignement. Les flèches noires désignent les sites de clivage primaire et secondaire présumés. Ils ont été déterminés par analogie avec les sites connus dans *P*.*falciparum* (27, 28). Des lignes verticales et des flèches horizontales localisent les limites des quatre régions qui ont été étudiées. La région 4 correspond à la séquence codant pour la p19 de *P*.*cynomolgi*. Des sites de glycosylation sont encadrés et les cystéines conservées sont soulignées. Dans la partie inférieure de la **Figure 4** sont indiqués les pourcentages identité entre les deux isolats de *P.vivax* et *P. cynomolgi*.

La construction recombinante PcMSP1ₚ₁₉S contient de l'ADN correspondant aux 8 paires de bases de la séquence « leader » et les 32 premiers acides aminés de MSP1 de *Plasmodium vivax* de Met₁ à Asp₃₂ (isolat Belem ; Del Portillo et al. 1991. P.N.A.S. 88, 4030.) suivis par un GluPhe, dû au site EcoR1 faisant la liaison des deux fragments. Le tout est suivi par la séquence codant pour le MSP1ₚ₁₉ de *Plasmodium cynomolgi* (souche Ceylon) de Lys₂₇₆ à Ser₃₈₀. La construction est terminée par deux codons stop de TAA. Cette construction donne lieu à une protéine recombinante qui est sécrétée dans le surnageant de culture des cellules infectées.

### Purification de la protéine recombinante PfMSP1p19 par chromatographie d'immunoaffinité avec un anticorps monoclonal reconnaissant spécifiquement la p19 de Plasmodium falciparum.

La résine de chromatographie a été préparée en liant 70 mg d'un anticorps monoclonal (obtenu à partir d'un hybridome G17.12 déposé à la CNCM (Paris, France) le 14 février 1997 sous le n°I-1846 ; cet hybridome G17.12 a été construit à partir du myélome X63 Ag8 653 produisant des IgG 2a/k reconnaissant la p19 de *P*.*falciparum*) à 3g de CNBr-Sepharose 4B activé (Pharmacia) par des méthodes standards détaillées dans le mode d'emploi fourni par Pharmacia. Les surnageants de culture contenant le PfMSP1p19 soluble ont été incubés en batch avec la résine de chromatographie pendant 16 heures à 4°C. La colonne a été lavée une fois avec 20 volumes de 0.05% NP40, 0.5M NaCl, PBS ; une fois avec 5 volumes de PBS et une fois avec 2 volumes de 10 mM phosphate de sodium, pH 6.8. L'élution a été effectuée avec 30 ml de 0.2 M glycine, pH 2.2. L'éluat a été neutralisé avec 1 M phosphate de sodium, pH 7.7 puis concentré par ultrafiltration et dialysé contre du PBS. Pour la purification du PfMSP1p19 ancré, toutes les solutions de lavage et élution contenaient en supplément 0.1 % 3-(Dimethyl-dodecylammonio)-propane sulfonate (Fluka).

### Essai de vaccination de MSP1 recombinant de Plasmodium vivax (p42 et p19) chez le singe écureuil Saimiri sciureus.

Cet essai de vaccination a été fait chez des *Saimiri sciureus boliviensis* mâles de 2 à 3 ans, non splénectomisés. Trois singes ont été injectés 3 fois par voie intramusculaire à 3 semaines d'intervalle avec un mélange d'environ 50 à 100µg chacun, de PvMSP1ₚ₄₂ et ₚ₁₉ soluble recombinant (19), purifié par immunoaffinité. L'adjuvant de Freund complet et incomplet était utilisé comme suit : 1^{ère} injection : 1:1 FCA/FIA ; 2^{éme} injection 1:4 FCA/FIA ; 3^{ème} injection : FIA. Ces compositions d'adjuvant étaient mélangées par la suite 1:1 avec l'antigène en PBS. Les cinq singes contrôles recevaient l'antigène glutathione-S-transferase (GST) produit dans *E*.*coli* selon le même protocole. L'infection d'épreuve était effectuée en injectant 2.10⁶ hématies infectées avec une souche adaptée de *Plasmodium vivax* (Belem) 2.5 semaines après la dernière injection. La protection a été évaluée en déterminant les parasitémies journalières chez tous les animaux par examen des frottis colorés avec giemsa.

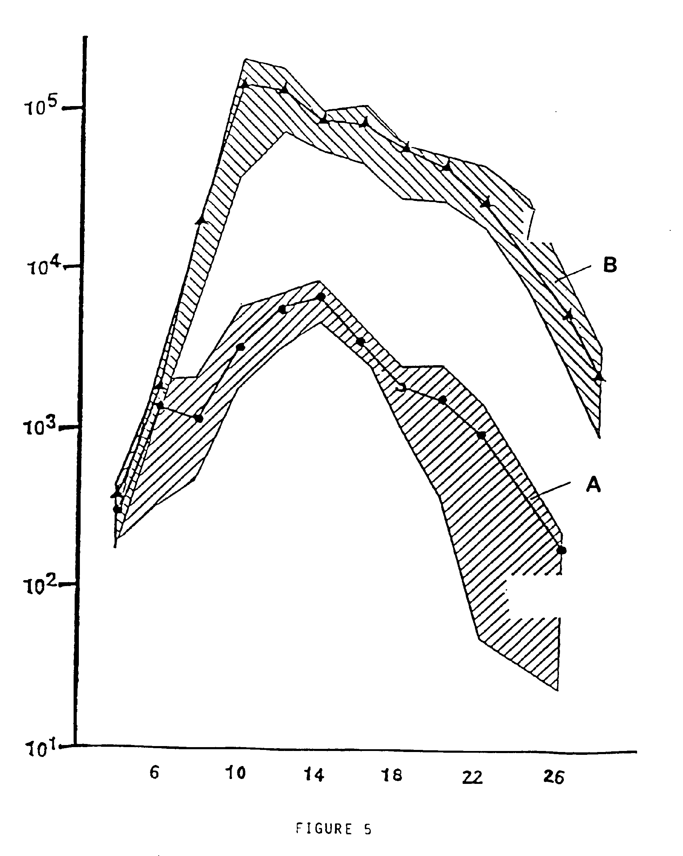

Les courbes de la **Figure 5** sont représentatives de la variation de la parasitémie mesurée en nombre d'hématies parasitées par microlitre de sang (sur l'axe des ordonnées à l'échelle logarithmique) en fonction du temps écoulé après l'infection (en jours). La courbe A correspond aux valeurs moyennes observées chez les trois singes vaccinés, la courbe B ; les valeurs moyennes chez cinq singes témoins.

De l'examen de la figure découle une très forte réduction de la parasitémie sous l'effet de la vaccination.

### Essai de vaccination de MSP1 recombinant de Plasmodium cynomolgi (p42 et p19) chez le singe toque, Macaca sinica.

Quinze singes capturés ont été utilisés comme suit : **(1)** 3 animaux injectés avec 100 µg PcMSP1ₚ₄₂ soluble ; 3 animaux injectés avec 35 µg (1^{ère} injection) ou 50 µg (2^{ème} et 3^{ème} injections) PcMSP1p₁₉ soluble ; **(3)** 3 animaux injectés avec un mélange de PcMSP1ₚ₄₂ et ₚ₁₉ ; **(4)** 3 animaux injectés avec l'adjuvant plus PBS ; **(5)** 3 animaux non injectés. L'adjuvant de Freund complet et incomplet a été utilisé selon le protocole décrit ci-dessus. Les injections on été faites par voie intramusculaire à 4 semaines d'intervalle. L'infection d'épreuve était faite en injectant 2.10⁵ hématies infectées avec *Plasmodium cynomolgi* 4 semaines après la dernière injection. La protection a été évaluée en déterminant les parasitémies journalières chez tous les animaux en examinant les parasitémies avec giemsa. Les parasitémies ont été classées comme négatives uniquement après comptage de 400 champs de frottis. Les parasitémies sont exprimées en pourcentage d'hématies parasitées.

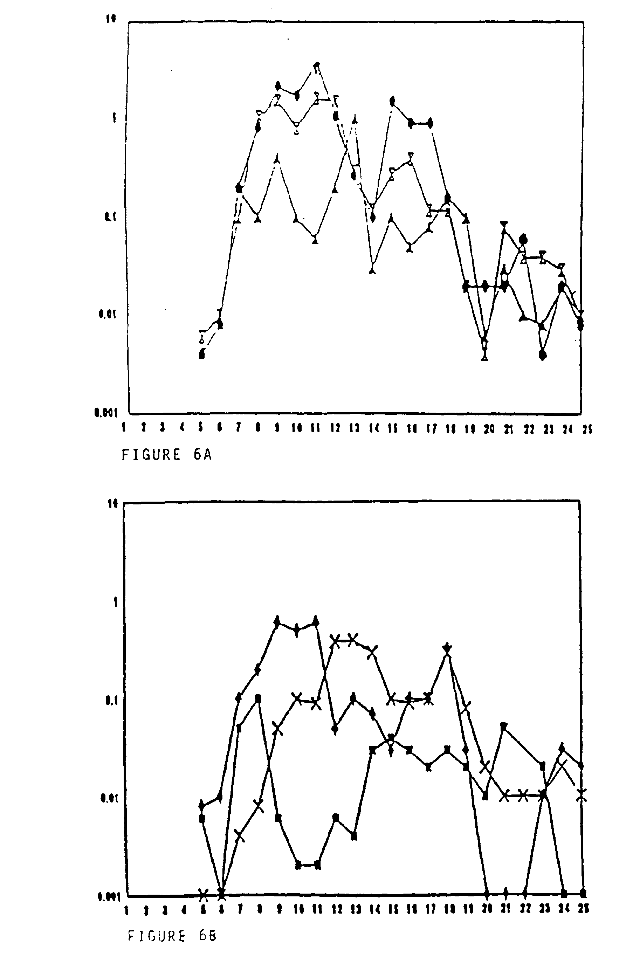

Les **Figures 6A-6G** sont illustratives des résultats obtenus. Dans chacune d'elles apparaissent les parasitémies (exprimées en pourcentages d'hématies parasitées sur l'axe des ordonnées à l'échelle logarithmique) observées chez les animaux d'épreuve en fonction des temps après l'infection (en jours sur les axes des abscisses).

Les résultats concernent :
- dans la figure 6A ; des animaux contrôles non vaccinés ;
- la **figure 6B** concerne des animaux qui avaient reçu une solution saline contenant en outre l'adjuvant de Freund ;

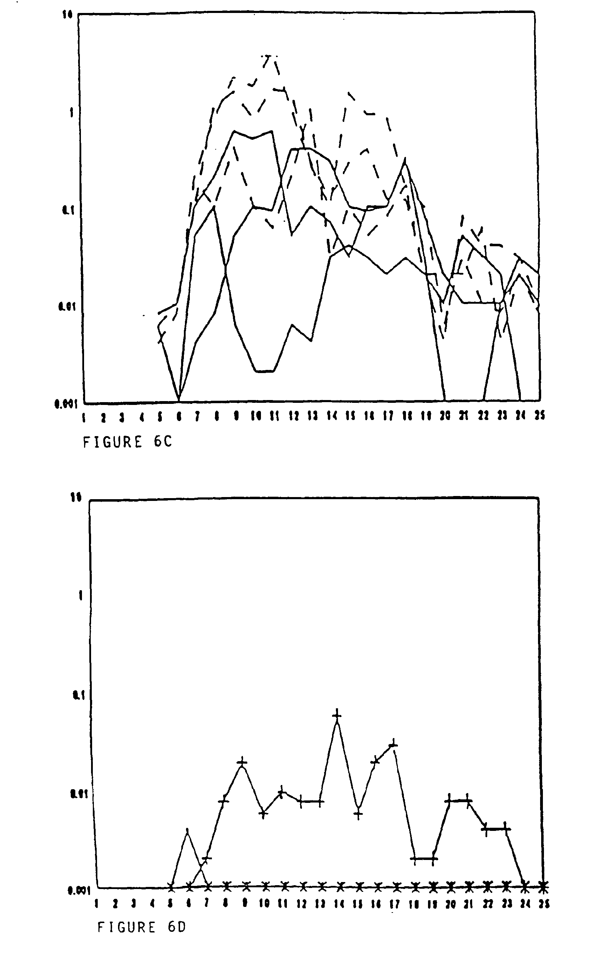

- la **figure 6C** est une superposition des figures 6A et 6B, dans le but de faire apparaître les résultats relatifs résultant de l'administration de l'adjuvant de Freund aux animaux (les variations ne sont évidemment pas significatives) ;
- la **figure 6D** fournit les résultats obtenus à l'issue d'une vaccination avec la p42 ;

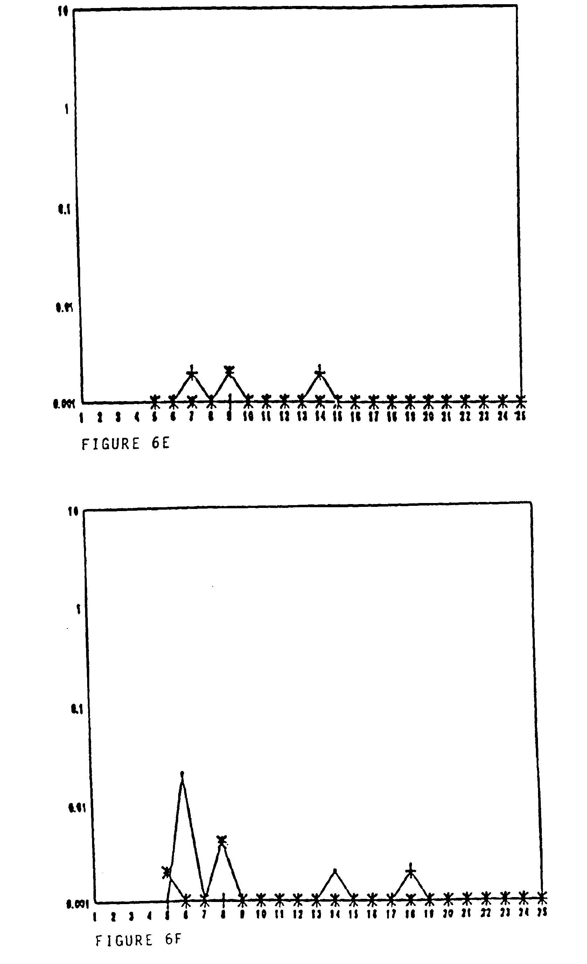

- la **figure 6E** concerne des animaux vaccinés avec la seule p19 ;
- enfin, la **figure 6F** concerne les animaux vaccinés avec un mélange de p19 et p42.

La p42 induit certes un certain niveau de protection. Mais comme en témoignent les figures 6E et 6F, la protection conférée par la p19 recombinante selon l'invention est considérablement améliorée.

On peut formuler l'hypothèse que l'amélioration de la protection résulte d'un clivage secondaire de la p42 qui s'accompagne du dévoilement de cystéine libre qui forme, par la suite, des ponts disulfure intermoléculaires donnant lieu à des multimères du p19 très caractéristiques de cette forme dans les protéines recombinantes des trois espèces testées.

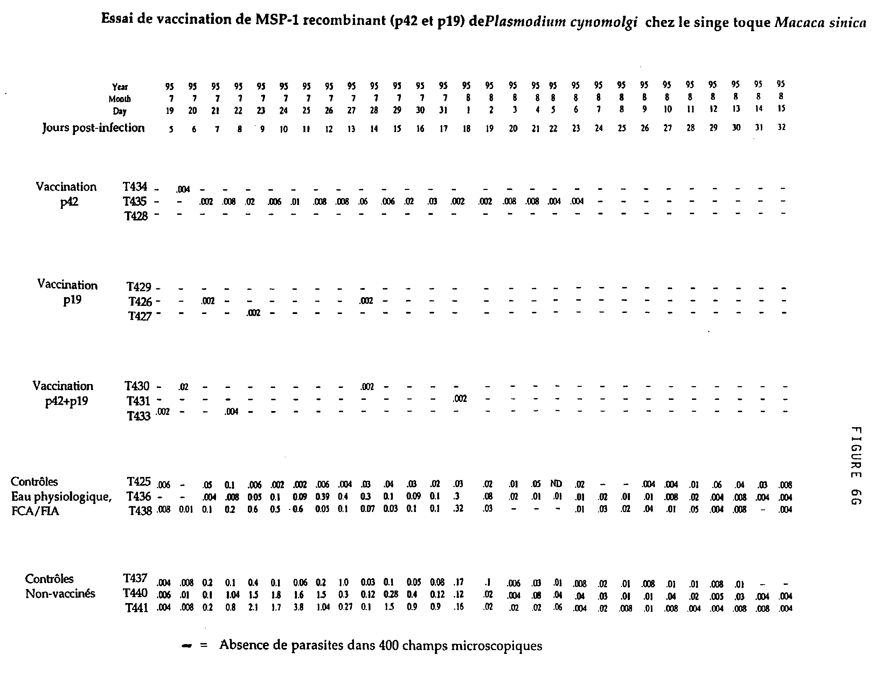

Les chiffres utilisés pour élaborer les graphiques (6A-6F) sont précisés dans la **Figure 6G****.**

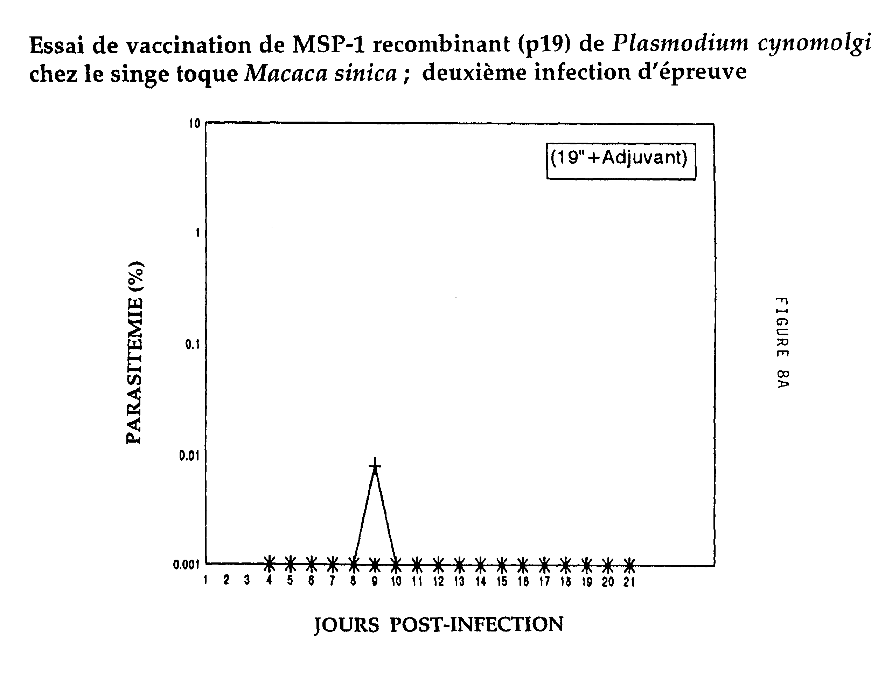

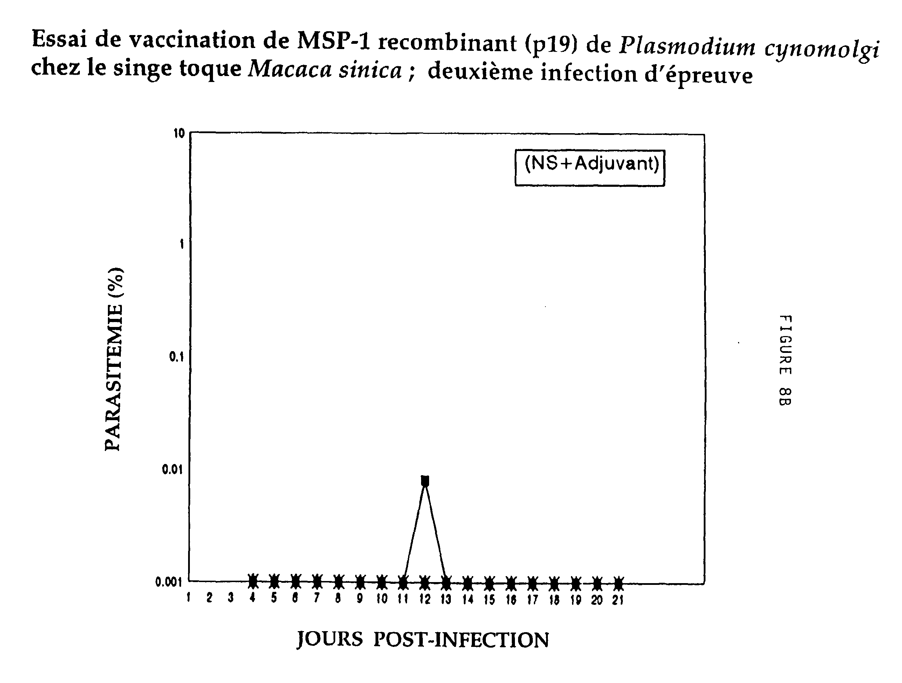

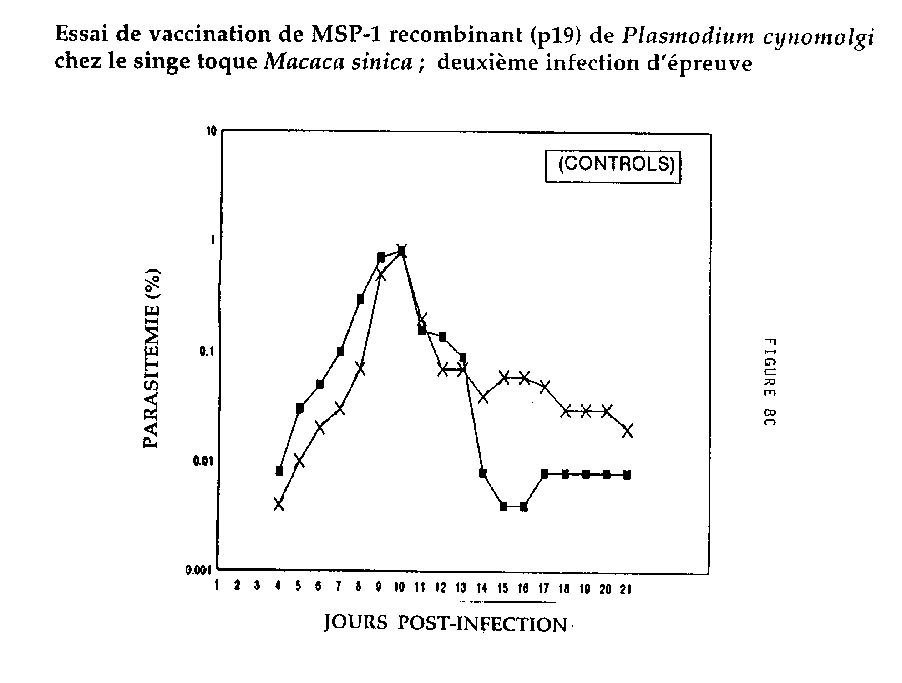

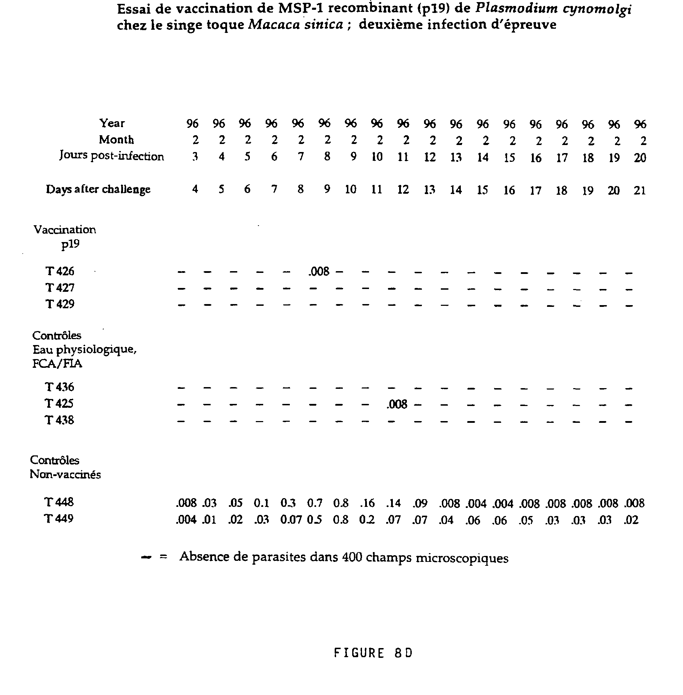

### Essai de vaccination P.cynomolgi/singe toque ; deuxième infection d'épreuve sur singes vaccinés avec p19 seule et contrôles (Figures 8)

Six mois plus tard, sans autre immunisation, les 3 singes ayant reçu le MSP-1 p19 seule avec FCA/FIA (Figure 6E) et les 3 singes ayant reçu une solution saline contenant l'adjuvant de Freund (Figure 6B) ainsi que 2 nouveaux singes naïfs non vaccinés ont subi une nouvelle infection d'épreuve par injection de 1.10⁶ hématies infectées avec *Plasmodium cynomolgi*. La protection a été évaluée en déterminant les parasitémies journalières chez tous les animaux en examinant les frottis avec du giemsa. Les parasitémies ont été classées comme négatives uniquement après comptage de 400 champs de frottis. Les parasitémies sont exprimées en pourcentage d'hématies parasitées (les chiffres utilisés pour élaborer les graphiques 8A-C sont précisés dans la **Figure 8D**). Les six animaux immunisés qui avaient subi une infection d'épreuve six mois auparavant n'avaient pas de parasitémie détectable sauf pour 1 animal dans chaque groupe qui a présenté une parasitémie à 0.008% pendant 1 jour (**Figures 8A** **et** **8B**)**.** Les deux contrôles naïfs montrent une parasitémie classique avec un maximum de 0.8% et pendant 21 jours (**Figure 8C**). Donc, les 3 animaux vaccinés avec le MSP-1 p19 étaient aussi protégés six mois plus tard que les 3 contrôles qui présentaient une infection complète classique après le première infection d'épreuve, malgré une absence ou un très faible parasitémie après la première infection d'épreuve. Ces résultats suggèrent que la durée de protection du p19 est au moins de six mois.

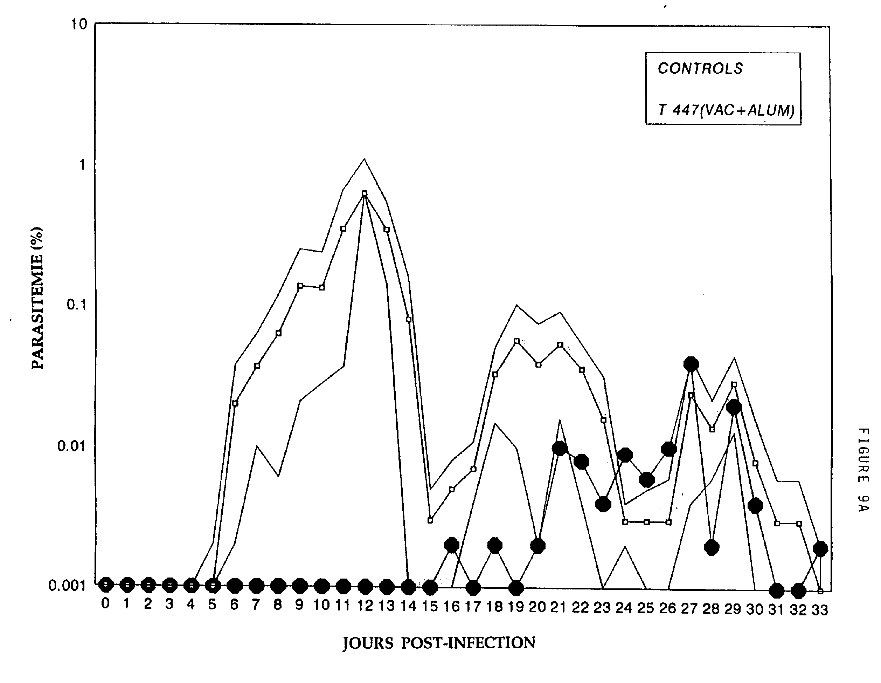

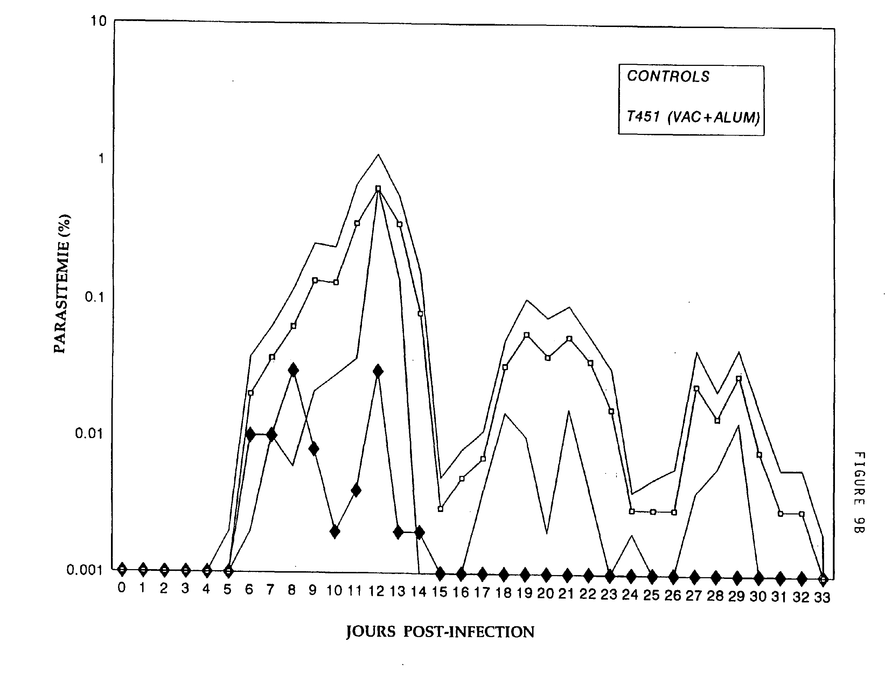

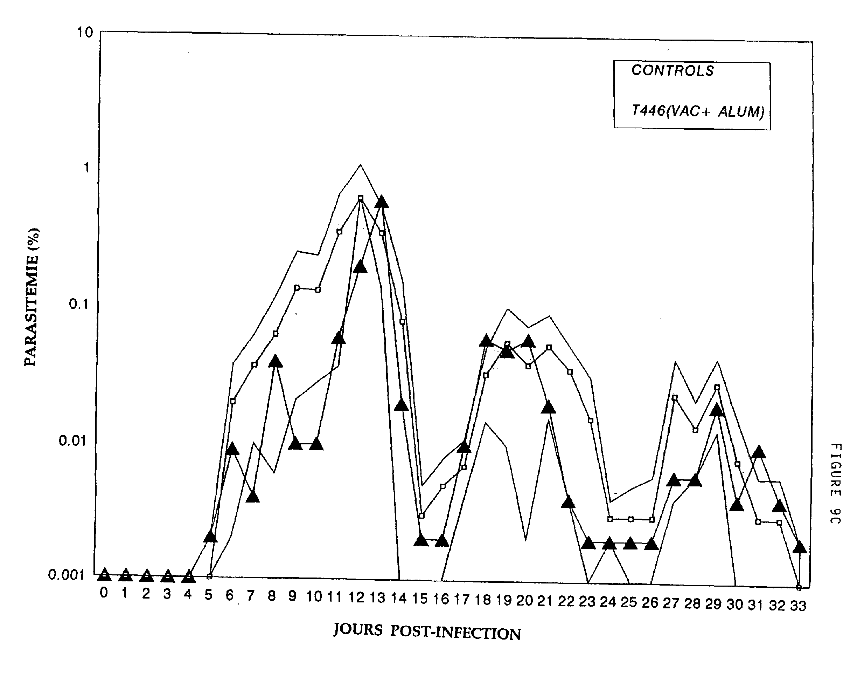

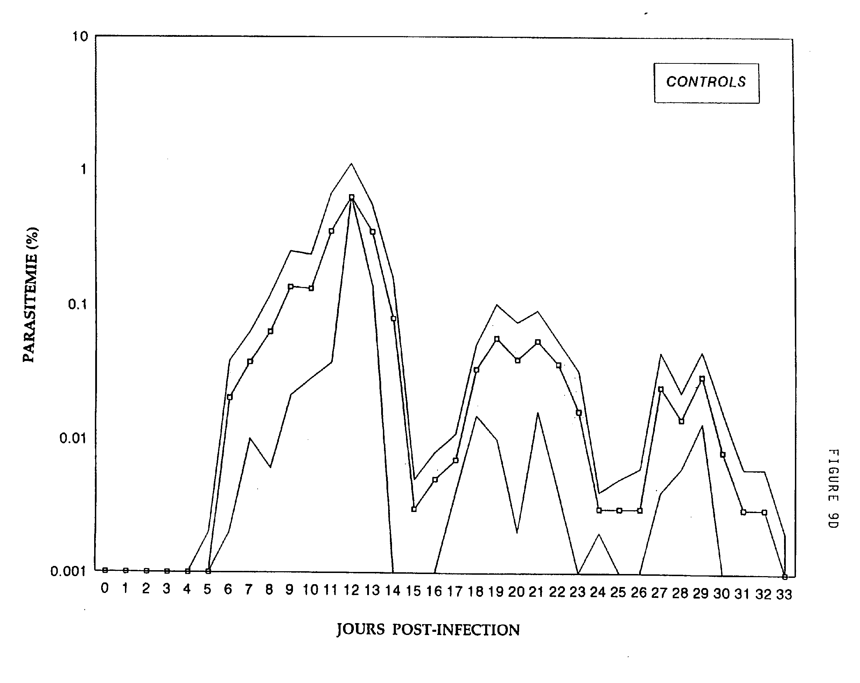

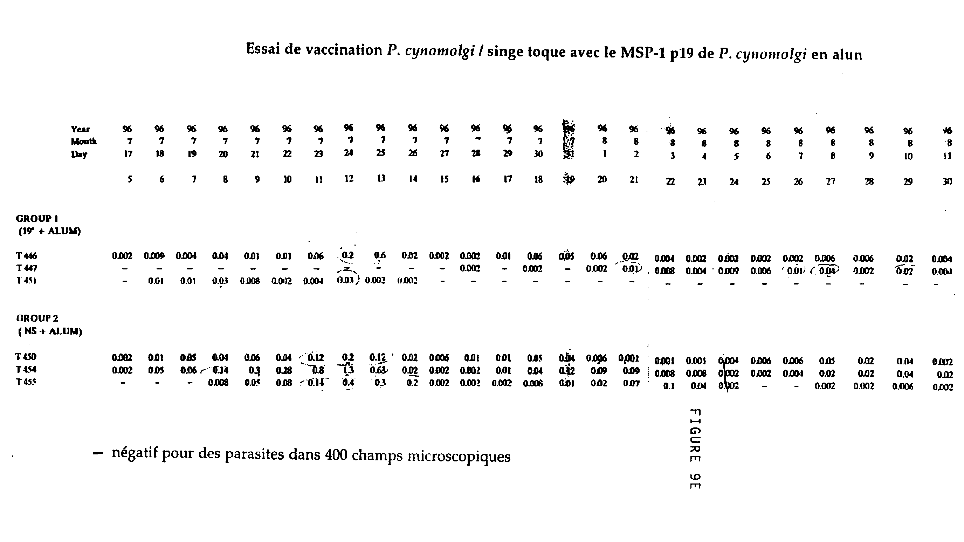

### Essai de vaccination avec p19 en association avec alun dans le système P.cynomolgi/singe toque (Figures 9)

Les résultats positifs de protection précédents ont été obtenus en utilisant l'adjuvant complet (FCA) ou incomplet (FIA) de Freund. Cependant, le seul adjuvant admis actuellement chez l'homme est l'alun. Pour cette raison, nous avons fait un essai de vaccination avec le MSP-1 p19 de *P. cynomolgi* chez le singe toque en présence d'alun comme adjuvant. Six singes capturés ont été utilisés comme suit: (1) 3 animaux injectés avec 4 doses de 50 mg MSP-1 p19 recombinant de *P*. *cynomolgi* avec 20 mg d'alun (2) 3 animaux injectés 4 fois avec de l'eau physiologique et 10 mg de alun. Les injections ont été faites par voie intramusculaire à 4 semaines d'intervalle. L'infection d'épreuve était faite en injectant 2.10⁵ hématies infectées avec *P. cynomolgi* 4 semaines après la dernière injection. La protection a été évaluée en déterminant les parasitémies journalières chez tous les animaux en examinant les frottis avec le giemsa. Les parasitémies ont été classées comme négatives uniquement après comptage de 400 champs de frottis. Les parasitémies sont exprimées en pourcentage d'hématies parasitées. Les résultats de cette expérience sont les suivants: 2 des 3 singes immunisés avec le p19 recombinant en alun avaient environ 30 fois moins de parasitémie totale pendant la durée de l'infection (**Figures 9A** **et** **9B**) que les 3 singes contrôles immunisés avec de l'eau physiologique et de l'alun (**Figure 9D**) après l'infection d'épreuve. Le troisième singe immunisé avec p19 (**Figure 9C**) n'était pas très différent des contrôles. Pour l'essai de vaccination de *Plasmodium cynomolgi* p19 chez le singe toque, *Macaca sinica*, décrit dans la Figure 9, les chiffres utilisés pour élaborer les graphiques (9A-9D) sont précisés (**Figure 9E**). Bien que ces résultats soient un peu moins spectaculaires que les précédents (Figures 6, 8) ; c'est la première fois qu'une protection significative est observée pour MSP-1 recombinante en alun.

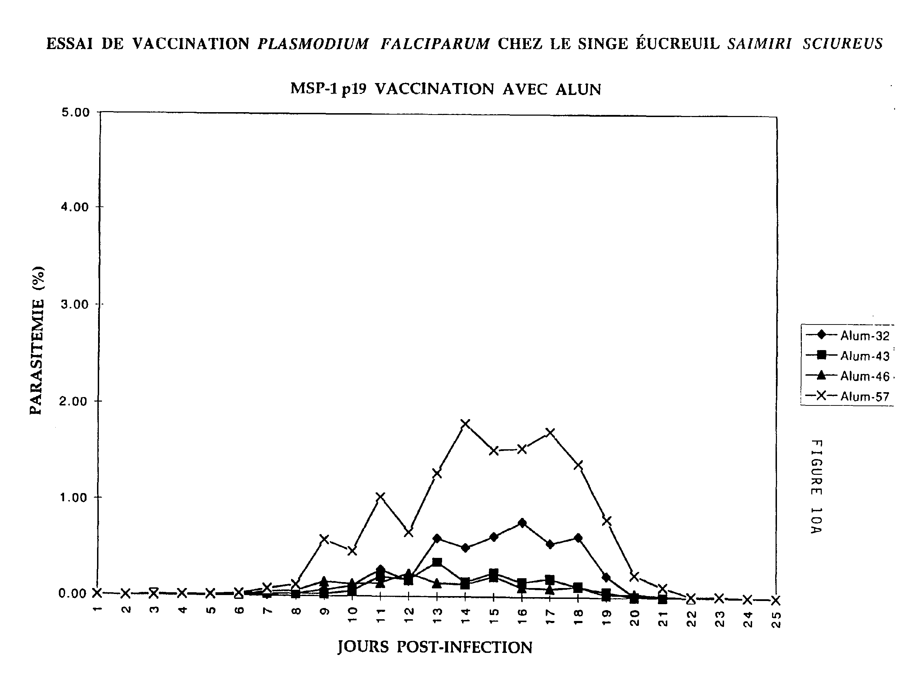

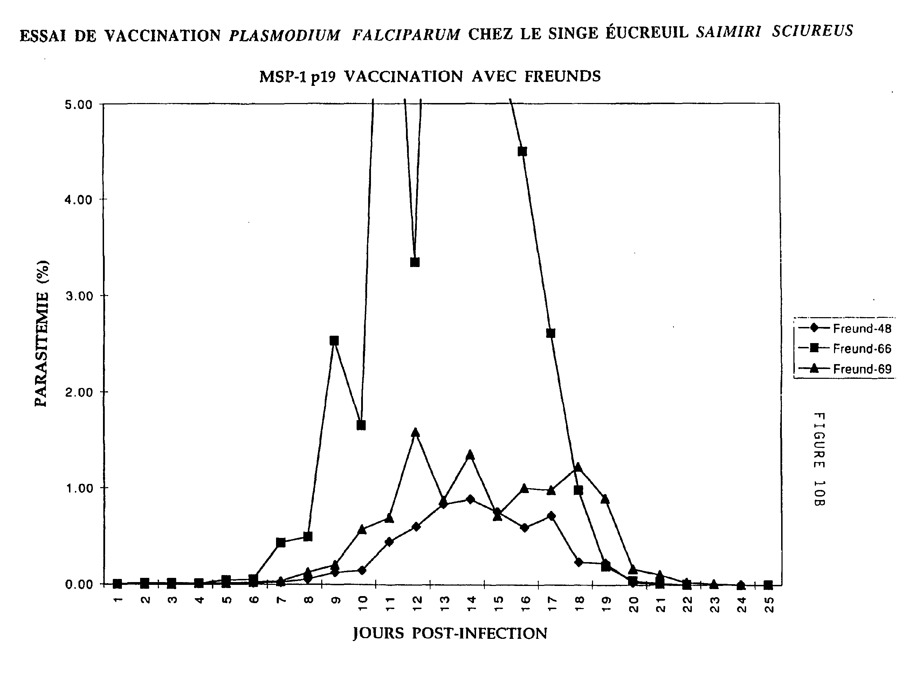

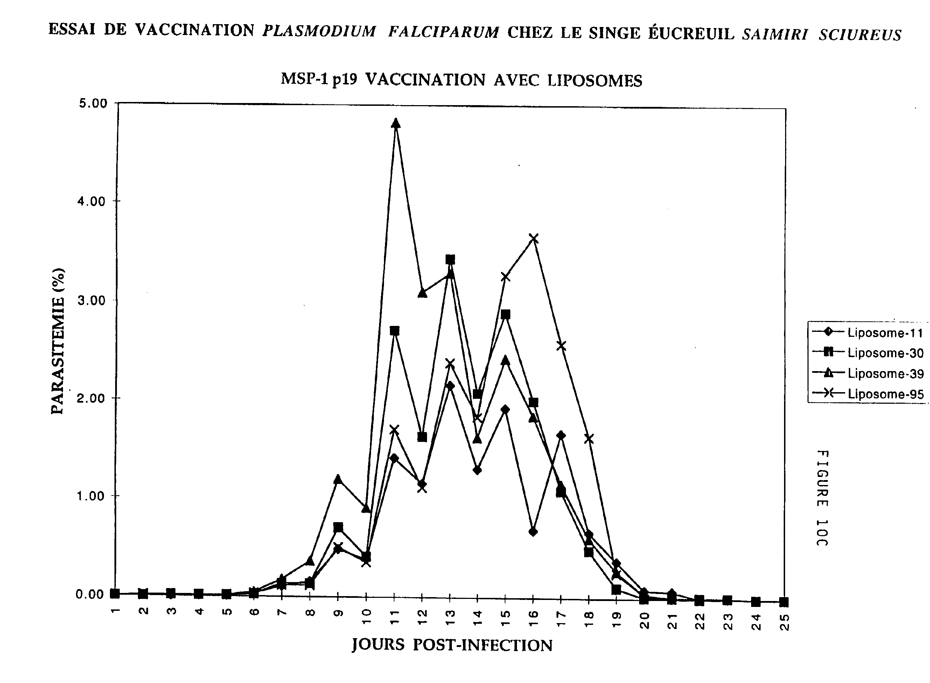

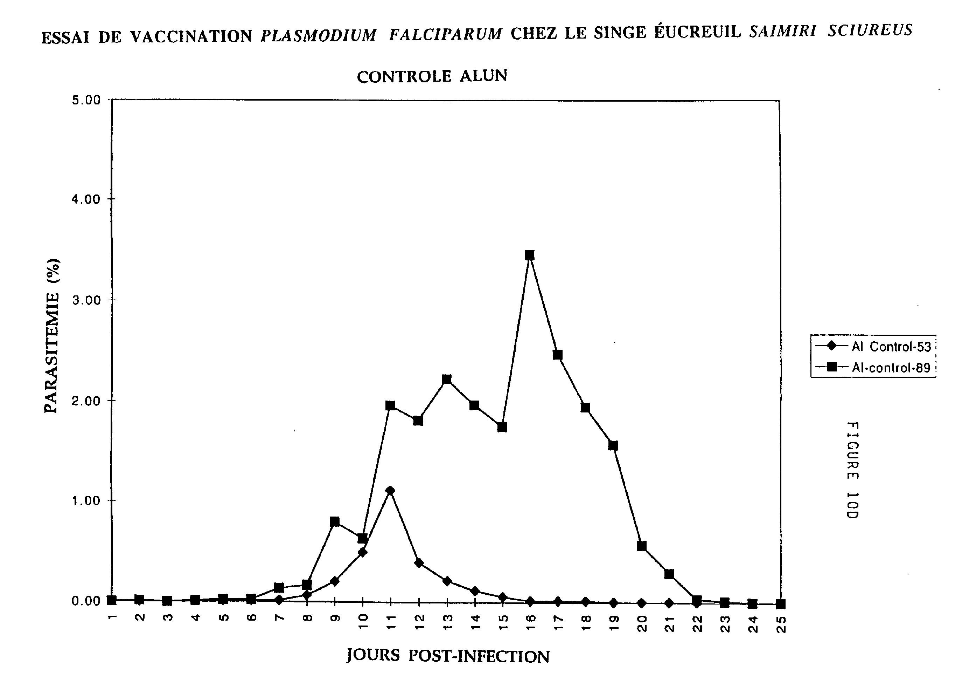

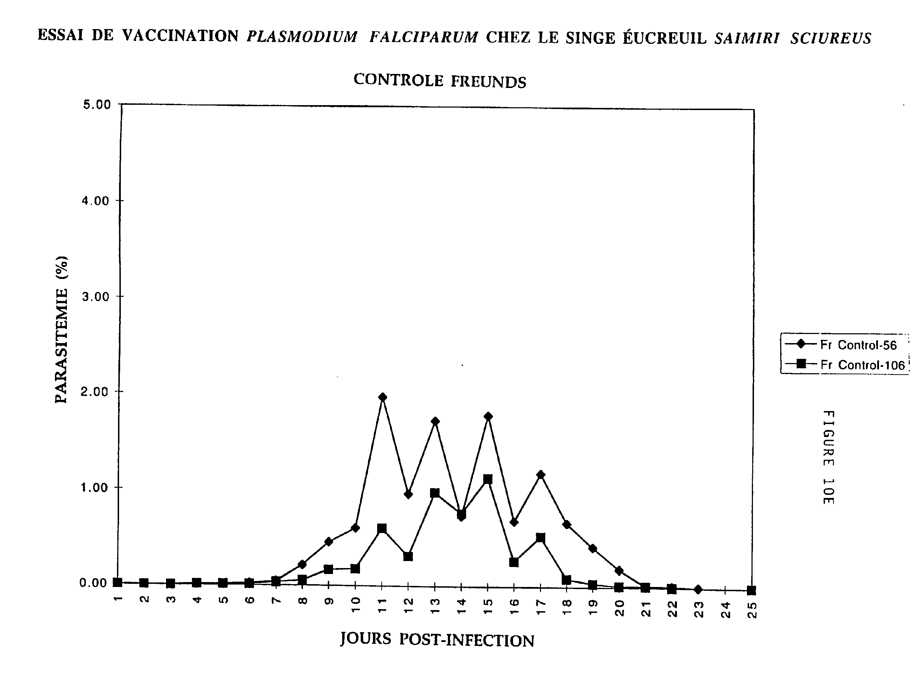

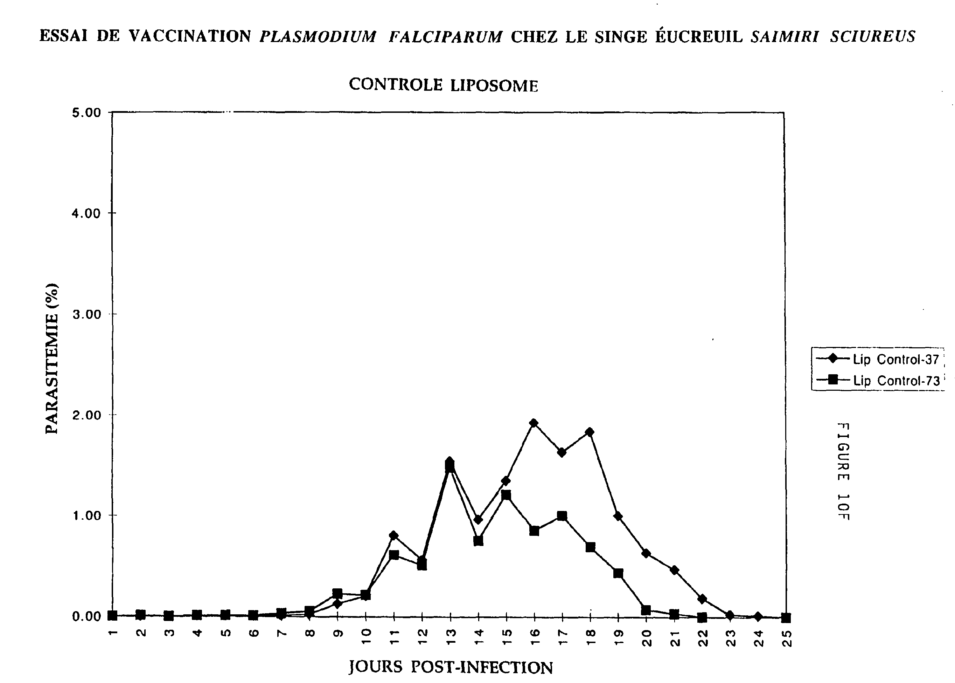

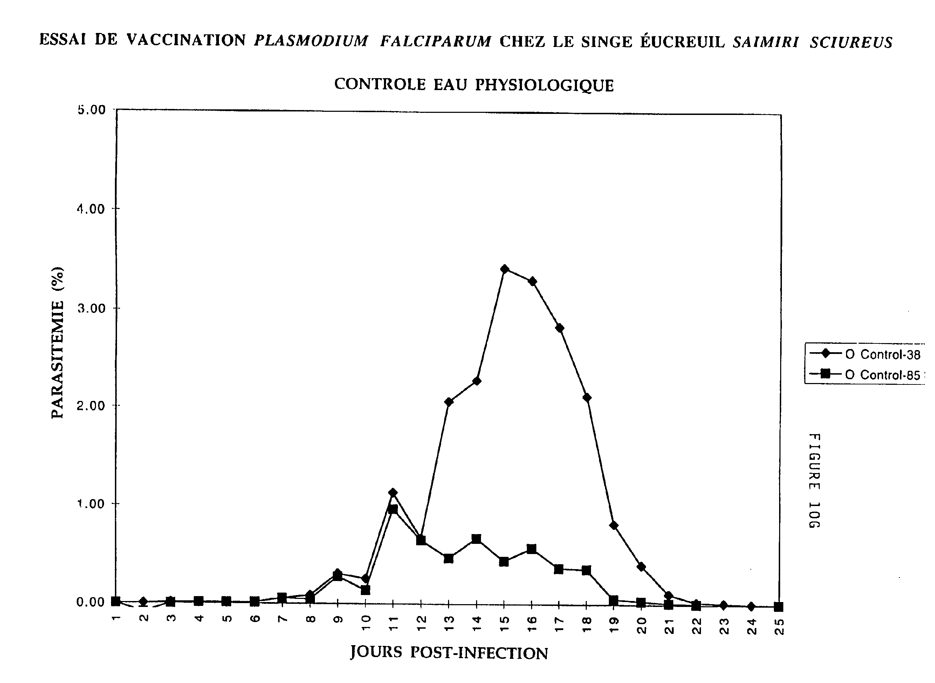

**Figure 10** : Essai de vaccination avec une p19 recombinante de *Plasmodium falciparum* chez le singe écureuil.

Vingt singes *Saïmiri sciureus guyanensis* (singe écureuil) d'environ 3 ans élevés en captivité ont été utilisés comme suit: (1) 4 animaux injectés avec 50 mg de Pf MSP-1 p19 soluble en présence d'adjuvant de Freund comme suit: 1^{ère} injection: 1:1 FCA/FIA; 2^{ème} injection: 1:4 FCA/FIA; 3^{ème} injection: FIA Ces compositions d'adjuvant ont ensuite été mélangées avec l'antigène en PBS 1:1. (2) 2 animaux contrôles recevaient l'adjuvant de Freund comme décrit pour (1) avec uniquement PBS; (3) 4 animaux injectés avec 50 mg de Pf MSP-1 p19 soluble en présence de 10 mg d'alun (Alu-Gel-S, Serva); (4) 2 animaux contrôles recevaient 10 mg d'alun avec uniquement PBS; (5) 4 animaux injectés avec environ 50-100 mg Pf MSP-1 p19 ancré GPI reconstitués en liposomes comme suit: 300 mmoles de cholestérol et 300 mmoles de phosphatidyl choline étaient séchés sous vide et resuspendus en 330 mM N-octylglucoside en PBS avec 1.4 mg de Pf MSP-1 p19,GPI. Cette solution avait été dialysée contre PBS avec des Bio-Beads SM-2 adsorbant (Bio-Rad) et les liposomes ainsi formés étaient concentrés par centrifugation et resuspendus en PBS. La 1^{ère} injection était faite avec des liposomes frais maintenus à 4°C et les 2^{éme} et 3^{éme} injections étaient faites avec des liposomes ayant été congelés pour conservation; (6) 2 animaux injectés avec des liposomes contrôles faits de la même façon en absence de l'antigène p19, GPI, comme décrit pour (5); (7) 2 animaux injectés avec de l'eau physiologique. Trois injections ont été faites par voie intramusculaire à 4 semaines d'intervalle. L'infection d'épreuve était faite en injectant 1.10⁶ hématies infectées avec *Plasmodium falciparum*. La protection a été évaluée en déterminant les parasitémies journalières chez tous les animaux en examinant les frottis avec giemsa. Les parasitémies sont exprimées en pourcentage d'hématies parasitées. Les résultats de cet essai de vaccination sont exposés dans les **Figures 10****, A-G.**

Les groupes immunisés avec le p19 en adjuvant de Freund ou en liposome ont démontré des parasitémies semblables aux groupes contrôles après une infection d'épreuve (un animal (numéro 29) vacciné avec p19 en adjuvant de Freund est mort quelques jours après l'infection d'épreuve pour des raisons indépendantes de la vaccination (crise cardiaque)). Des irrégularités dans l'administration de l'antigène dans ces 2 groupes (mauvaise émulsion de Freund, liposomes congelés) ne permettent pas d'évaluer de façon complète la signification de ces résultats. Dans le groupe alun 2 animaux ont montré des parasitémies totales pendant la durée de l'infection environ 4 fois moins importantes que les contrôles, I animal environ 3 fois moins importante et 1 animal était semblable aux contrôles. Cette expérience est un peu difficile à interpréter à cause de la variabilité dans les contrôles, probablement due à la souche de parasite utilisée pour l'infection d'épreuve qui n'aurait pas été assez adaptée au modèle *Saimiri* non spénectomisé mis au point que récemment à Cayenne. Néanmoins, l'effet réel, quoiqu'imparfait, avec l'alun est encourageant dans la mesure où nos antigènes semblent être les seules versions recombinantes MSP-1 de *P. falciparum* qui, pour l'instant, ont démontré une certaine efficacité en association avec l'alun.

### Essai de vaccination avec une p19 recombinante de Plasmodium falciparum chez le singe écureuil (même essai que Figures 10)

Des singes élevés en captivité ont été injectés avec 1 ml d'inoculum par voie intramusculaire 2 fois à 4 semaines d'intervalle comme suit : (**1)** 4 animaux injectés avec 50 µg de PfMSP1p19 soluble en présence d'adjuvant de Freund comme suit: I^{ère} injection 1:1 FCA/FIA; 2^{ème} injection 1:4 FCA/FIA; et mélangés par la suite 1:1 avec l'antigène en PBS; **(2)** 4 animaux injectés avec 50 µg de PfMSP1p19 soluble en présence de 10 mg d'alun; **(3)** 4 animaux injectés avec environ 50 µg de PfMSP1p19 ancré GPI reconstitués en liposomes composés 1:1 en molarité de cholestérol et phosphatidyl choline. Les animaux ont été saignés 17 jours après la deuxième injection.

Les globules rouges provenants d'un singe écureuil avec 30% de parasitémie due à *P. falciparum* (avec des formes mûres en majorité) ont été lavés en PBS et le culot était dilué 8 fois en présence de 2% SDS et 2% dithiothreitol et chauffé à 95° avant d'être chargé sur un gel de polyacrylamide de 7.5% (gel de séparation) et 4% (gel de stacking) (haut du gel). Après transfère en nitrocellulose l'analyse par immunoblot (immunoempreinte) a été fait avec des antisera comme suit: **(1)** pool d'antisera des 4 singes vaccinés avec PfMSP1p19 soluble en adjuvant de Freund dilué au vingtième ; **(2)** pool d'antisera des 4 singes vaccinés avec PfMSP1p19 soluble en adjuvant alun dilué au vingtième; **(3)** pool d'antisera des 4 singes vaccinés avec PfMSP1p19 ancré en liposomes dilué au vingtième ; **(4)** l'anticorps monoclonal, qui réagit avec un épitope linéaire de PfMSP1p19, à 50 µg/ml ; **(5)** pool d'antisera SHI90 provenant d'un vingtaine de singes infectés à répétition par *P. falciparum* et devenus réfractaires à toute infection ultérieure de *P. falciparum*, dilué au cinq centième ; **(6)** pool d'antisera des singes naïfs (n'ayant jamais été exposés à *P. falciparum*) dilué au vingtième.

Les résultats montrent que les 3 pools d'antisera des singes vaccinés avec le PfMSP1p19 réagissent de façon importante et spécifique avec des complexes de très haut poids moléculaire (se trouvant de façon diffuse dans le gel de stacking) et présents dans des extraits de parasite contenant davantage de formes mûres. Ces résultats confortent l'hypothèse de la présence d'une agrégation spécifique du MSP1p19 *in vivo* comportant des épitopes qui sont reproduits dans les molécules recombinantes PfMSP1p19 synthétisées dans le système baculovirus, en particulier celles en forme d'oligomère.

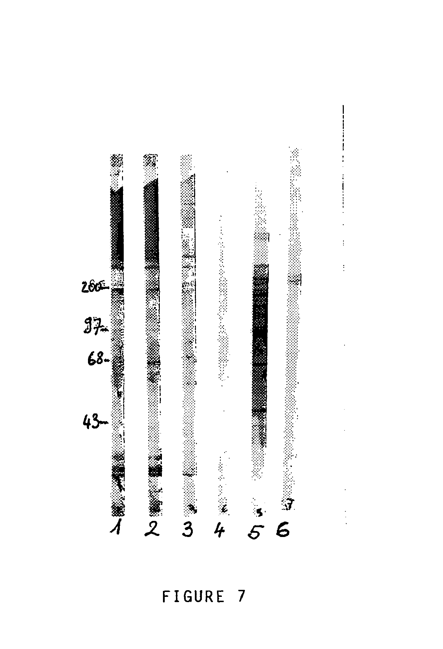

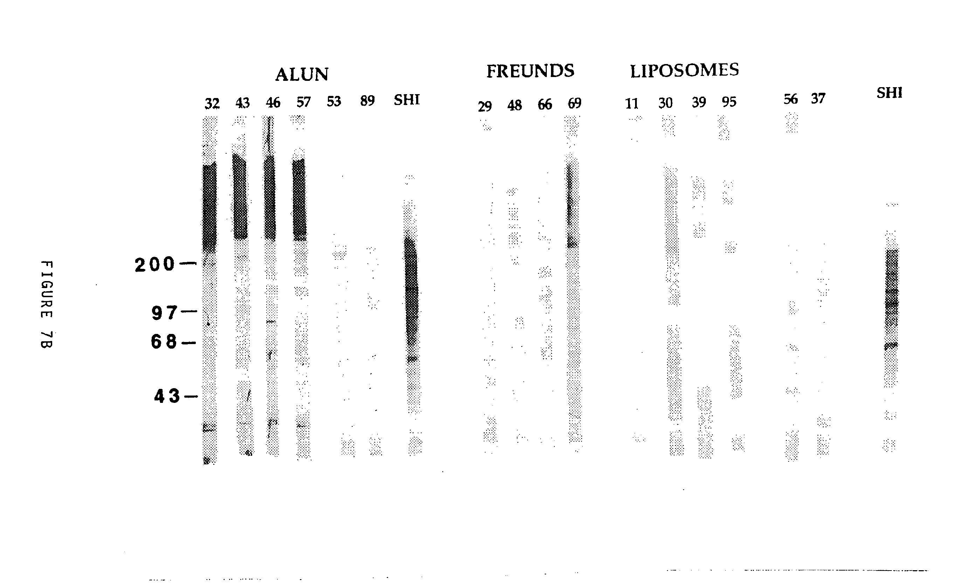

La **figure 7** illustre également ces résultats. Elle se rapporte aux immunoempreintes produites sur gel. Les trois premières colonnes du gel illustrent la réponse in vivo de singes à des injections de p19 [(1) avec l'adjuvant de Freund, (2) avec de l'alun, (3) sous forme de liposome] et notamment l'existence de complexes de haut poids moléculaire confortant l'hypothèse de l'agrégation in vivo de p19 sous forme d'oligomère, spécifique du stade de maturation (quand p42 est coupé en p19 et p33).

Cet essai de vaccination comprend également une troisième injection identique aux précédentes. L'injection avec l'adjuvant de Freund comprend uniquement du FIA.

Il y a deux animaux contrôles pour chaque groupe, à savoir : 2 animaux contrôles injectés avec PBS et l'adjuvant de Freund ; 2 animaux contrôles injectés avec PBS et alun ; 2 animaux contrôles injectés avec liposomes sans protéine ; et deux animaux injectés avec PBS sans adjuvant. La protection est évaluée comme décrit ci-dessus.

**Figure 7B****:** Les données pour cette figure sont dérivées de l'essai de vaccination de *P. falciparum l* singe écureuil (Figure 10 ci-après). Les chiffres correspondent aux singes individuels notés dans la Figure 10. Les techniques et méthodes pour cette figure sont les mêmes que pour la Figure 7 sauf que l'antisérum individuel de chaque singe noté a été testé après trois injections le jour de l'infection d'épreuve et l'antisérum SHI a été dilué 1:250. Les résultats montrent que l'antisérum des 4 singes vaccinés avec le p19 et alun réagissent de façon importante et spécifique avec des complexes de très haut poids moléculaire tandis que les singes des autres groupes vaccinés avec le p19 et l'adjuvant de Freund ou des liposomes ne montrent qu'une faible réactivité avec ces complexes. Puisque les singes vaccinés avec p19 et alun ont aussi été le mieux protégés, cette réactivité avec les complexes de haut poids moléculaire paraît indiquer un effet protecteur, et cela malgré qu'un singe dans le groupe alun n'était pas protégé par rapport aux contrôles et qu'un autre ne l'était que partiellement.

L'invention concerne naturellement d'autres applications, par exemple celles exposées ci-après en rapport avec certains des exemples, lesquels ne présentent aucun caractère limitatif.

### Thérapeutique

La molécule recombinante PfMSP1p19 peut être utilisée pour produire des anticorps spécifiques éventuellement utilisables par transfert passif dans un but de thérapeutique adaptée au paludisme sévère dû à *P. falciparum* avec risque de mortalité.

### Diagnostic

Les molécules recombinantes PvMSP1 p42 et PvMSP1p19 et PfMSP1p19 dérivées de baculovirus peuvent et ont été utilisées pour produire des anticorps monoclonaux spécifiques murins. Ces anticorps, en combinaison avec des antisera polyclonaux anti MSP1p19 provenant d'une autre espèce telle que le lapin ou la chèvre, peuvent être à la base d'un test de diagnostic semi-quantitatif pour le paludisme et capable de distinguer entre un paludisme dû à P. falciparum, qui peut être mortel, et un paludisme dû à P. vivax, qui n'est généralement pas mortel. Le principe de ce test serait de piéger et quantifier toute molécule de MSP1 contenant la partie p19 dans le sang.

Dans ce cadre, les avantages de la molécule MSP1p19 sont les suivants:
(i) elle est à la fois extrêmement bien conservée au sein d'une même espèce et suffisamment divergente entre des espèces différentes pour permettre de produire facilement des réactifs spécifiques d'espèce. Aucune réaction croisée n'a été observée entre les anticorps dérivés de PfMSP1p19 et PvMSP1p19:
(ii) la fonction du MSP1p19, bien que non connue avec précision, semble être suffisamment importante pour que cette molécule ne varie pas de façon significative ou soit délétée sans effet létal pour le parasite ;
(iii) c'est un antigène majeur se trouvant sur tous les mérozoïtes et donc, il doit être, en principe, détectable même à basse parasitémie et proportionnellement à la parasitémie ;
(iv) puisque les molécules recombinantes de MSP1p19 dérivées de , baculovirus semblent reproduire davantage la structure native de MSP1p19, les anticorps produits contre ces protéines seraient bien adaptés à un usage diagnostique.

Les microorganismes identifiés ci-dessous ont été déposés suivant la règle 6.1. du Traité de Budapest à la date du 01 février 1996, sous les numéros suivants :

| Références d'identification | Numéros d'enregistrement |
|---|---|
| PvMSP1p19A | I - 1659 |
| PvMSP1p19S | I - 1660 |
| PfMSP1p19A | I - 1661 |
| PfMSP1p19S | I - 1662 |
| PcMSP1p19S | I - 1663 |

La présente divulgation concerne également l'utilisation de ces anticorps, alors de préférence préalablement fixés sur un support solide (par exemple pour chromatographie d'affinité), pour la purification de peptides du type p19 initialement contenus dans un mélange.

La purification fait alors intervenir une mise en contact de ce mélange avec l'anticorps, la dissociation du complexe antigène-anticorps et la récupération du peptide de type p19 purifié.

La présente divulgation concerne également des compositions de vaccin, comprenant également des mélanges de protéines ou de fragments, notamment des mélanges du type :
- p19 de *P*.*falciparum* et p19 de *P*.*vivax*,
- p19 de *P*.*falciparum* et p42 de *P*.*falciparum*, celle-ci étant le cas échéant dépourvue de ses régions les plus hypervariables,
- p19 de *P. vivax* et p42 de *P*.*vivax*, celle-ci étant le cas échéant dépourvue de ses régions les plus hypervariables,
- p19 de *P*.*falciparum* et p42 de *P*.*falciparum*, celle-ci étant le cas échéant dépourvue de ses régions les plus hypervariables, et p19 de *P.vivax* et p42 de *P*.*vivax*, celle-ci étant le cas échéant dépourvue de ses régions les plus hypervariables.

On entend dans le cas présent définir les régions les plus hypervariables comme la région II ou la région II et la région III en partie ou dans sa totalité, la partie de la région III préférablement délétée étant celle qui est juxtaposée à la région II (la partie conservée se situant du côté C-terminal de la p33, proche de la p19). Les régions II et III sont illustrées en figure 4.

La présente divulgation pas limitée à la production de vaccin humain. Elle s'applique tout autant à la production de compositions de vaccin vétérinaire mettant en oeuvre les protéines ou antigènes correspondants dérivés de parasites infectieux pour des mammifères et produits dans les mêmes conditions. Il est en effet connu que des infections du même type, la babésiose, apparaissent aussi chez les bovins, les canins et les équins un des antigènes des espèces Babésia présente une forte homologie conformationnelle (notamment les deux domaines « EFG-like » et richesse en cystéine) et fonctionnelle avec une partie protéique de MSP-1 [(36), (37) et (38)].

Des exemples de vaccins vétérinaires utilisant un antigène soluble contre de tels parasites on été décrits (39).

Il va sans dire que les p19 mises en oeuvre dans ces mélanges peuvent également donner lieu à toutes les modifications dont il a été question dans ce qui précède, lorsqu'elle était prise en considération de façon isolée.

### RÉFÉRENCES

**(1)** Holder, J.A. et al. (1982) « Biosynthesis and processing of a Plasmodium falciparum schizont antigen recognized by immune serum and a monoclonal antibody ». J. Exp. Med. 156 :1528-1538.
**(2)** Howard, R. et al. (1984) « Localization of the major Plasmodium falciparum glycoprotein on the surface of mature intracellular trophozoïtes and schizonts ». Mol. Biochem. Parasitol. 11 : 349-362.
**(3)** Pirson, P. et al. (1985) « Characterization with monoclonal antibodies of a surface antigen of Plasmodium falciparum merozoïtes », J. Immunol. 134 : 1946-1951.
**(4)** Aley, S.B. et al. (1987) « Plasmodium vivax: Exoerythrocytic schizonts recognized by monoclonal antibodies against blood-stage schizonts ». Exp. Parasitol. 64 : 188-194.
**(5)** Holder, A.A. (1988) « The precursor to major merozoïte surface antigen: structure and role in immunity ». Prog. Allergy 41 : 72-97.
**(6)** Cooper, J.A. (1993) « Merozoïte surface antigen-1 of Plasmodium ». Parasitol. Today 9 : 50-54.
**(7)** Holder, A.A., et al. (1987) « Processing of the precursor to the major merozoïte antigens of Plasmodium falciparum » Parasitology 94 : 199-208.
**(8)** Lyon, J.A. et al. (1986) « Epitope map and processing scheme for the 195 000-dalton surface glycoprotein of Plasmodium falciparum merozoïtes deduced from cloned overlapping segments of the gene ». Proc. Natl. Acad. Sci. USA 83: 2989-2993.
**(9)** Blackman, M.J. et al. (1992) « Secondary processing of the Plasmodium falciparum merozoïte surface protein-1 (MSP1) by calcium-dependent membrane-bound serine protease: shedding of MSP133 as a noncovalently associated complex with other fragments of the MSOP1 ». Mol. Biochem. Parasitol. 50 : 307-316.
**(10)** Haldar, K., et al. (1985) « Acylation of a Plasmodium falciparum merozoïte surface antigen via sn-1,2-diacyl glycerol. ». J. Biol. Chem. 260 : 4969-4974.
**(11)** Braun Breton, C. et al. (1990) « Glycolipid anchorage of Plasmodium falciparum surface antigens ». Res. Immunol. 141 : 743-755.
**(12)** Kumar, S. et al. (1995) « Immunogenicity and in vivo Efficacy of Recombinant Plasmodium falciparum Merozoïte surface protein-1 in Aotus Monkeys ». Molecular Medicine, Vol. 1, 3 : 325-332.
**(14)** Longacre, S. et al. (1994) « Plasmodium vivax merozoïte surface protein 1 C-terminal recombinant proteins in baculovirus ». Mol. Biochem. Parasitol. 64 : 191-205.
**(15)** McBride, J.S. et al. (1987) « Fragments of the polymorphic Mr 185 000 glycoprotein from the surface of isolated Plasmodium falciparum merozoïtes form an antigenic complex ». Mol. Biochem. Parasitol. 23 : 71-84.
**(16)** Blackman, M.J. et al. (1990) « A single fragment of a malaria merozoïte surface protein remains on the parasite during red cell invasion and is the target of invasion-inhibiting antibodies ». J. Exp. Med. 172 : 379-382.
**(17)** Kaslow, D.C. et al. (1994) « Expression and antigenicity of Plasmodium falciparum major merozoïte surface protein (MSP119) variants secreted from Saccharomyces cerevisiae ». Mol. biochem. Parasitol. 63 ; 283-289.
**(18)** Chang, S.P., et al. (1992) « A carboxyl-terminal fragment of Plasmodium falciparum gp195 expressed by a recombinant baculovirus induces antibodies that completely inhibit parasite growth ». J. Immunol. 149 : 548-555
**(19)** Longacre, S. (1995) « The Plasmodium cynomolgi merozoïte surface protein 1 C-terminal sequence and its homologies with other Plasmodium species ». Mol. Biochem. Parasitol. 74 :105-111.
**(20)** Del Portillo, H.A., et al. (1990) « Primary structure of the merozoïte surface antigen 1 of Plasmodium vivax reveals sequences conserved between different Plasmodium species ». Proc. Natl. Acad. Sci. USA 88 : 4030-4034.
**(21)** Gibson, H.L., et al. (1992) « Structure and expression of the gene for Pv200, a major blood-stage surface antigen of Plasmodium vivax ». Mol. biochem. Parasitol. 50 : 325-334.
**(22)** Dissanaike, A.S., et al. (1965) « Two new malaria parasites, Plasmodium cynomolgi ceylonensis sub sp. nov. and Plasmodium fragile sp. nov. from monkeys in Ceylon ». Ceylon Journal of Medical Science 14 : 1-9.
**(23)** Cochrane, A.H., et al. (1986) « Further studies on the antigenic diversity of the circumsporozoïte proteins of the Plasmodium cynomolgi complex. ». Am. J. Trop. Med. Hyg. 35 : 479-487.
**(24)** Naotunne, T. de S., et al. (1990) « Plasmodium cynomolgi: serum-mediated blocking and enhancement of infectivity to mosquitos during infections in the natural host, Macaca sinica ». Exp. Parasitol. 71, 305-313.
**(25)** Ihalamulla, R.L. et al. (1987) « Plasmodium vivax: isolation of mature asexual stages and gametocytes from infected human blood by colloidal silica (Percoll) gradient centrifugation ». Trans. R. Soc. Trop. Med. Hyg. 81 : 25-28.
**(26)** Kimura, E., et al. (1990) « Genetic diversity in the major merozoite surface antigen of Plasmodium falciparum: high prevalence of a third polymorphic form detected in strains derived in strains derived from malaria patients ». Gene 91 : 57-62.
**(27)** Heidrich, H.-G., et al. (1989) « The N-terminal amino acid sequences of the Plasmodium falciparum (FCBI) merozoïte surface antigen of 42 and 36 kilodalton, both derived from the 185-195 kilodalton precursor ». Mol. Biochem. Parasitol. 34 : 147-154.
**(28)** Blackman, M.J., et al. (1991) « Proteolytic processing of the Plasmodium falciparum merozoïte surface protein-1 produces a membrane-bound fragment containing two epidermal growth factor-like domains ». Mol. Biochem. Parasitol. 49 : 29-34.
**(29)** Adams, J.M. et al. (1992) « A family of erythrocyte binding proteins of malaria parasites ». Proc. Natl. Acad. Sci, 89:7085-7089.
**(30)** Sim B.K.L. (1995) « EBA-175: An erythrocyte-binding ligand of Plasmodium falciparum ». Parasitology Today, vol. II, n°6:213-217.
**(31)** Sim B.K.L. (1994) « Receptor and ligand domains for invasion of erythrocytes by Plasmodium falciparum ». Science, 264:1941-1944.
**(32)** Davies, A. et al. (1993), Biochem J. 295 (Pt3) : 889-896. « Expression of the glycosylphosphatidylinositol-linked complement-inhibiting protein CD59 antigen in insect cells using a baculovirus vector ».
**(33)** Haziot A. et al, (1994) J. Immunol. 152: 5868. « Recombinant soluble CD14 Inhibits LPS-Induced Tumor Necrosis Factor & Production by Cells in Whole Blood ».
**(34)** Chang, S.P. et al., (1988) « Plasmodium falciparum : gene structure and hydropathy profile of the major merozoite surface antigen (gp195) of the Uganda-Palo Alto isolate. Exp. Parasitol. 67 : 1-11.
**(35)** Holder, A.S. et al. (1985) « Primary structure of the precursor to the three major surface antigens of Plasmodium falciparum merozoites, Nature 317 : 270-273.
**(36)** G. Bourdoiseau et al. (mai 1995) « Les babésioses bovines », Le point vétérinaire, vol.27, n°168**.**
**(37)** P. Bourdeau et al. (mai 1995) « La babésiose canine à Babesia canis », Le point vétérinaire, vol.27, n°168**.**
**(38)** C. Soulé (mai 1995) « Les babésioses équines », Le point vétérinaire, vol.27, n°168
**(39)** T.P.M. Schetters et al. (1995) « Vaccines against Babesiosis using Soluble Parasite Antigens », Parasitology Today, vo1.11, n°12**.**
**(40)** P.A. Burghaus et al. (1996) « Immunization of Aotus nancymai with Recombinant C Terminus of Plasmodium falciparum Merozoite Surface Protein 1 in Liposomes and Alun Adjuvant Does Not Induce Protection against a Challenge Infection », Infection and Immunity, 64:3614-3619.
**(41)** S.P. Chang, et al. (1996) « A Recombinant Baculovirus 42-Kilodaiton C-Terminal Fragment of Plasmodium falciparum Merozoite Surface Protein 1 Protects Aotus Monkeys against Malaria », Infection and Immunity, 64: 253-261.
**(42)** L.H. Miller et al. (1997) « The Need for Assays Predictive of Protection in Development of Malaria Bloodstage Vaccines », Parasitology Today, vol.13, n°2:46-47.

L'invention concerne également les hybridomes sécréteurs d'anticorps spécifiques reconnaissant sélectivement la p19 d'une protéine MSP-1 de la forme mérozoïte d'un parasite du type *Plasmodium* infectieux pour l'homme autre que *Plasmodium vivax* et ne reconnaissant pas *Plasmodium vivax*.

En particulier, ces hybridomes sécrètent des anticorps monoclonaux qui reconnaissent pas la p19 de *Plasmodium vivax et* qui reconnaissent spécifiquement la p19 de *Plasmodium falciparum*.

L'invention concerne également un hybridome **caractérisé en ce qu**'il produit un anticorps spécifique qui reconnaît spécifiquement la p19 de *P*.*vivax* et la p19 de *P*.*cynomolgi*. Un hybridome F10-3 a été construit à partir du myélome X63 Ag8 653 produisant des IgG 2b/k reconnaissant la glycoprotéine p42 de *Plasmodium vivax*.

## Revendications

1. ADN codant un fragment polypeptidique ou une partie de fragment polypeptidique,
lequel fragment ou partie étant apte à induire une réponse immune capable d'inhiber une parasitémie due à un parasite *Plasmodium falciparum*, **caractérisé en ce que** ledit ADN comprend une séquence nucléotidique synthétique qui code :
- un fragment C-terminal de 19 kilodaltons (p19) de la protéine 1 de surface (MSP-1) de la forme mérozoïte d'un *Plasmodium falciparum*, ce fragment C-terminal restant normalement ancré à la surface du parasite au terme de sa pénétration dans des érythrocytes humains, à l'occasion d'un cycle infectieux,
ou
- une partie du fragment p19 qui contient au moins l'une des deux régions EGF contenues dans le fragment p19 sauvage de la MSP-1 de la forme mérozoïte d'un *Plasmodium falciparum*,
ladite séquence nucléotidique synthétique ayant en outre une teneur en G et C comprise entre 40 et 60% de la totalité des nucléotides dont elle est constituée.

2. ADN selon la revendication 1, **caractérisé en ce que** ladite séquence nucléotidique synthétique est dépourvue de la séquence correspondant à la séquence d'ancrage de la protéine native.

3. ADN selon la revendication 1 ou 2, **caractérisé en ce que** ladite séquence nucléotidique synthétique code pour une forme soluble de fragment p19 ou partie de fragment p19.

4. ADN selon l'une quelconque des revendications 1 à 3, **caractérisé en ce que** ladite séquence nucléotidique synthétique a une teneur en G et C d'au moins 50% de la totalité des nucléotides dont elle est constituée.

5. ADN selon l'une quelconque des revendications 1 à 4, **caractérisé en ce qu'**il comprend en outre, en amont de ladite séquence nucléotidique synthétique, une séquence nucléotidique qui code une séquence polypeptidique comprenant moins de 50 résidus de la partie C-terminale du fragement de 33 kitodaltons (p33) de MPS-1.

6. ADN selon l'une quelconque des revendications 1 à 5, **caractérisé en ce qu'**il comprend en outre une séquence nucléotidique codant un peptide signal, en amont de l'extrémité 5'-terminale de ladite séquence nucléotidique synthétique, ce peptide signal étant susceptible d'être reconnu en tant que signal dans un système baculovirus.

7. ADN selon la revendication 6, **caractérisé en ce que** ledit peptide signal est un peptide signal de MSP-1 de Plasmodium.

8. ADN selon la revendication 7, **caractérisé en ce que** ledit peptide signal est un peptide signal de MSP-1 de *Plasmodium falciparum*.

9. ADN selon l'une quelconque des revendications 1, 2, 3, 4, 6, 7, **caractérisé en ce que :**
ladite séquence nucléotidique synthétique code un fragment C-terminal de 19 kilodaltons (p19) de la protéine 1 de surface (MSP-1) de la forme mérozoïte d'un *Plasmodium falciparum* de Asn1613 à Ser1705,
et **en ce qu'**en amont de l'extrémité 5'-terminale de cette séquence nucléotidique synthétique, l'ADN comprend:
- les 8 paires de bases de la séquence leader de la protéine 1 de surface de Plasmodium vivax, et
- une séquence codant les trente-deux acides aminés de la protéine 1 de surface de Plasmodium vivax de Met1 à Asp32,
et **en ce qu'**en aval de l'extrémité 3'-terminale de cette séquence nucléotidique synthétique, l'ADN comprend deux codons stop TAA.

10. ADN selon l'une quelconque des revendications 1, 6, 7, **caractérisé en ce que** :
ladite séquence nucléotidique synthétique code un fragment C-terminal de 19 kilodaltons (p19) de la protéine 1 de surface (MSP-1) de la forme mérozoïte d'un *Plasmodium falciparum* de Asn1613 à Ile1726,
et **en ce qu'**en amont de l'extrémité 5'-terminale de cette séquence nucléotidique synthétique, l'ADN comprend:
- les 8 paires de bases de la séquence leader de la protéine 1 de surface de Plasmodium vivax, et
- une séquence codant les trente-deux acides aminés de la protéine 1 de surface de Plasmodium vivax de Met1 à Asp32,
et **en ce qu'**en aval de l'extrémité 3'-terminale de cette séquence nucléotidique synthétique, l'ADN comprend deux codons stop TAA.

11. Vecteur baculovirus recombinant contenant, sous le contrôle d'un promoteur contenu dans ce vecteur et susceptible d'être reconnu par des cellules transfectables par ce vecteur :
- un ADN selon l'une quelconque des revendications 1 à 5, et une séquence nucléotidique codant un peptide signal, en amont de l'extrémité 5'-terminale de cet ADN, ce peptide signal étant susceptible d'être reconnu en tant que signal dans un système baculovirus, ou
- un ADN selon l'une quelconque des revendications 6 à 10.

12. Vecteur baculovirus selon la revendication 11, **caractérisé en ce qu'**il est choisi parmi le virus déposé à la CNCM sous le numéro I-1661, et le virus déposé à la CNCM sous le numéro I-1662.

13. Cellule d'insecte transfectée par un vecteur baculovirus selon la revendication 11 ou 12.

14. Fragment polypeptidique, ou partie de fragment polypeptidique, lequel fragment ou partie étant apte à induire une réponse immune capable d'inhiber une parasitémie due à un parasite *Plasmodium falciparum*, **caractérisé en ce que** ledit fragment ou partie de fragment est susceptible d'être :
- exprimé par un vecteur baculovirus selon la revendication 11 ou 12, ou
- produit par une cellule selon la revendication 13,
et **en ce que** ledit fragment ou partie de fragment
- comporte des épitopes conformationnels instables en milieu réducteur et constituant la majorité des épitopes reconnus par des antisérums humains formés contre *Plasmodium falciparum*, et
- est reconnu par des antisérums humains formés contre *Plasmodium falciparum* lorsqu'elle est à l'état non réduit ou dans un état réduit non irréversible, mais non reconnue ou peu reconnue par ces mêmes antisérums, lorsqu'elle est irréversiblement réduite.

15. Oligomère de fragments polypeptidiques ou de parties de fragments polypeptidiques selon la revendication 14.

16. Protéine comprenant un fragment polypeptidique ou partie de fragment polypeptidique selon la revendication 14.

17. Composition adaptée à la vaccination contre un parasite *Plasmodium falciparum*,
**caractérisée en qu'**elle comprend :
- un fragment polypeptidique ou partie de fragment polypeptidique selon la revendication 14, ou un oligomère selon la revendication 15, ou une protéine selon la revendication 16.

18. Procédé pour la production d'un fragment polypeptidique ou d'une partie de fragment polypeptidique, lequel fragment ou partie étant apte à induire une réponse immune capable d'inhiber une parasitémie due à un parasite *Plasmodium falciparum*, **caractérisé en ce qu'**il comprend le fait de :
insérer un ADN selon l'une quelconque des revendications 1 à 10 dans un vecteur baculovirus, de sorte à ce que ce vecteur puisse exprimer le fragment polypeptidique codé par cet ADN,
transfecter une cellule d'insecte par ce vecteur,
récolter le fragment polypeptidique ou partie de fragment polypeptidique produit par cette cellule.

## Claims

1. DNA coding for a polypeptide fragment or a portion of a polypeptide fragment, said fragment or portion being suitable for inducing an immune response which is capable of inhibiting a parasitemia due to a *Plasmodium falciparum* parasite, **characterized in that** said DNA comprises a synthetic nucleotide sequence which codes for:
• a 19 kilodalton (p19) C-terminal fragment of the surface protein 1 (MSP-1) of the merozoite form of a *Plasmodium falciparum*, said C-terminal fragment remaining normally anchored to the parasite surface at the end of its penetration phase into human erythrocytes in the event of an infectious cycle; or
• a portion of a p19 fragment which contains at least one of two EGF regions contained in the wild type p19 fragment of the MSP-1 of the merozoite form of a *Plasmodium falciparum*;
said synthetic nucleotide sequence also having a G and C content in the range of 40% to 60% of the totality of the nucleotides from which it is constituted.

2. DNA according to claim 1, **characterized in that** said synthetic nucleotide sequence is deprived of the sequence corresponding to the anchoring sequence for the native protein.

3. DNA according to claim 1 or claim 2, **characterized in that** said synthetic nucleotide sequence codes for a soluble form of p19 fragment or a portion of p19 fragment.

4. DNA according to any one of claims 1 to 3, **characterized in that** said synthetic nucleotide sequence has a G and C content of at least 50% of the totality of the nucleotides from which it is constituted.

5. DNA according to any one of claims 1 to 4, **characterized in that** it further comprises, upstream of said synthetic nucleotide sequence, a nucleotide sequence which codes for a polypeptide sequence comprising less than 50 residues of the C-terminal portion of the 33 kilodaltons (p33) fragment of MSP1-1.

6. DNA according to any one of claims 1 to 5, **characterized in that** it further comprises a nucleotide sequence coding for a signal peptide upstream of the 5'-terminal end of said synthetic nucleotide sequence, said signal peptide being capable of being recognized as a signal in a baculovirus system.

7. DNA according to claim 6, **characterized in that** said signal peptide is a signal peptide of MSP-1 of *Plasmodium.*

8. DNA according to claim 7, **characterized in that** said signal peptide is a signal peptide of MSP-1 of *Plasmodium falciparum.*

9. DNA according to any one of claims 1, 2, 3, 4, 6, 7, **characterized in that**: said synthetic nucleotide sequence codes for a 19 kilodaltons (p19) C-terminal fragment of the surface protein 1 (MSP-1) of the merozoite form of a *Plasmodium falciparum* from Asn1613 to Ser1705, and **in that** upstream of the 5'-terminal end of said synthetic nucleotide sequence, the DNA comprises:
• the 8 base pairs of the leader sequence of the surface protein 1 of *Plasmodium* vivax; and
• a sequence coding for the thirty-two amino acids of the surface protein 1 of *Plasmodium vivax* from Met1 to Asp32;
and **in that** downstream of the 3'-terminal end of said synthetic nucleotide sequence, the DNA comprises two TAA stop codons.

10. DNA according to any one of claims 1, 6, 7, **characterized in that**: said synthetic nucleotide sequence codes for a 19 kilodaltons (p19) C-terminal fragment of the surface protein 1 (MSP-1) of the merozoite form of a *Plasmodium falciparum* from Asn1613 to Ile1726, and **in that** upstream of the 5'-terminal end of said synthetic nucleotide sequence, the DNA comprises:
• the 8 base pairs of the leader sequence of the surface protein 1 of *Plasmodium* vivax; and
• a sequence coding for the thirty-two amino acids of the surface protein 1 of *Plasmodium vivax* from Met1 to Asp32; and **in that** downstream of the 3'-terminal end of said synthetic nucleotide sequence, the DNA comprises two TAA stop codons.

11. A recombinant baculovirus vector containing, under the control of a promoter contained in said vector and capable of being recognized by cells transfectable by said vector:
• a DNA according to any one of claims 1 to 5, and a nucleotide sequence coding for a signal peptide, upstream of the 5'-terminal end of said DNA, said signal peptide being capable of being recognized as a signal in a baculovirus system; or
• a DNA according to any one of claims 6 to 10.

12. A baculovirus vector according to claim 11, **characterized in that** it is selected from the virus deposited with the CNCM with accession number I-1661 and the virus deposited with the CNCM with accession number I-1662.

13. An insect cell transfected with a baculovirus vector according to claim 11 or claim 12.

14. A polypeptide fragment or a portion of a polypeptide fragment, said fragment or portion being suitable for inducing an immune response which is capable of inhibiting a parasitemia due to a *Plasmodium falciparum* parasite, **characterized in that** said fragment or fragment portion is capable of being:
• expressed by a baculovirus vector according to claim 11 or claim 12; or
• produced by a cell according to claim 13;
and **in that** said fragment or fragment portion
• comprises conformational epitopes which are unstable in a reducing medium and which constitute the majority of the epitopes recognized by human antiserums formed against *Plasmodium falciparum*; and
• is recognized by human antiserums formed against *Plasmodium falciparum* when it is in a non-reduced state or in a non-irreversible reduced state, but not recognized or recognized to only a slight extent by said antiserums when it is irreversibly reduced.

15. An oligomer of polypeptide fragments or portions of polypeptide fragments in accordance with claim 14.

16. A protein comprising a polypeptide fragment or a portion of a polypeptide fragment in accordance with claim 14.

17. A composition which is adapted for vaccination against a *Plasmodium falciparum* parasite, **characterized in that** it comprises:
• a polypeptide fragment or a portion of a polypeptide fragment according to claim 14, or an oligomer according to claim 15, or a protein according to claim 16.

18. A method for producing a polypeptide fragment or a portion of a polypeptide fragment, said fragment or portion being suitable for inducing an immune response which is capable of inhibiting parasitemia due to a *Plasmodium falciparum* parasite, **characterized in that** it comprises:
inserting a DNA in accordance with any one of claims 1 to 10 in a baculovirus vector, such that said vector can express the polypeptide fragment encoded by said DNA;
transfecting an insect cell with said vector;
harvesting the polypeptide fragment or portion of polypeptide fragment produced by said cell.

## Patentansprüche

1. DNA, die ein Polypeptidfragment oder einen Teil eines Polypeptidfragments kodiert, wobei das Fragment oder der Teil in der Lage ist, eine Immunantwort zu induzieren, die geeignet ist, eine Parasitämie infolge eines Parasiten *Plasmodium falciparum* zu hemmen,
**dadurch gekennzeichnet, dass** die DNA eine synthetische Nukleotidsequenz umfasst, die kodiert:
- ein C-terminales Fragment von 19 Kilodalton (p19) des Oberflächenproteins 1 (MSP-1) der Merozoitform von *Plasmodium falciparum,* wobei das C-terminale Fragment normalerweise bleibt am Ende seines Eindringens in menschliche Erythrozyten anlässlich eines Infektionszyklus auf der Oberfläche des Parasiten verankert, oder
- einen Teil des Fragments p19, der mindestens eine der beiden Regionen EGF enthält, die in dem Wildfragment p19 von MSP-1 der Merozoitform eines *Plasmodium falciparum* enthalten sind,
wobei die synthetische Nukleotidsequenz des Weiteren einen Gehalt an G und C zwischen 40 und 60 % aller Nukleotide, aus der sie gebildet ist, aufweist.

2. DNA gemäß Anspruch 1, **dadurch gekennzeichnet, dass** die synthethische Nukleotidsequenz nicht die Sequenz aufweist, die der Ankersequenz des nativen Proteins entspricht.

3. DNA gemäß Anspruch 1 oder 2, **dadurch gekennzeichnet, dass** die synthethische Nukleotidsequenz für eine lösliche Form des Fragments p19 oder einen Teil des Fragments p19 kodiert.

4. DNA gemäß einem der Ansprüche 1 bis 3, **dadurch gekennzeichnet, dass** die synthethische Nukleotidsequenz einen Gehalt an G und C von mindestens 50 % aller Nukleotide, aus denen sie gebildet ist, aufweist.

5. DNA gemäß einem der Ansprüche 1 bis 4, **dadurch gekennzeichnet, dass** sie des Weiteren aufwärts der synthethischen Nukleotidsequenz eine Nukleotidsequenz aufweist, die eine Polypeptidsequenz mit weniger als 50 Resten des C-terminalen Teils des Fragments von 33 Kilodalton (p33) von MSP-1 kodiert.

6. DNA gemäß einem der Ansprüche 1 bis 5, **dadurch gekennzeichnet, dass** sie des weiteren eine Nukleotidsequenz aufweist, die ein Peptidsignal aufwärts des 5'-terminalen Endes der synthetischen Nukleotidsequenz kodiert,
wobei das Peptidsignal geeignet ist, als Signal in einem Baculovirus-System erkannt zu werden.

7. DNA gemäß Anspruch 6, **dadurch gekennzeichnet, dass** das Peptidisgnal ein Peptidsignal von MSP-1 aus Plasmodium ist.

8. DNA gemäß Anspruch 7, **dadurch gekennzeichnet, dass** das Peptidisgnal ein Peptidsignal von MSP-1 aus *Plasmodium falciparum* ist.

9. DNA gemäß einem der Ansprüche 1, 2, 3, 4, 6, 7, **dadurch gekennzeichnet, dass:**
die synthetische Nukleotidsequenz ein C-terminales Fragment von 19 Kilodalton (p19) des Oberflächenproteins 1 (MSP-1) der Merozoitform von *Plasmodium falciparum* von Asn1613 bis Ser1705 kodiert,
und dadurch, dass aufwärts des 5'-terminalen Endes dieser synthetischen Nukleotidsequenz die DNA aufweist:
- die 8 Basenpaare der Leadersequenz des Oberflächenproteins 1 aus Plasmodium vivax, und
- eine Sequenz, die zweiunddreißig Aminosäuren des Oberflächenproteins 1 aus Plasmodium vivax von Met1 bis Asp32 kodiert,
und dadurch, dass abwärts des 3'-terminalen Endes dieser synthetischen Nukleotidsequenz die DNA zwei Stoppkodone TAA aufweist.

10. DNA gemäß einem der Ansprüche 1, 6, 7, **dadurch gekennzeichnet, dass:**
die synthetische Nukleotidsequenz ein C-terminales Fragment von 19 Kilodalton (p19) des Oberflächenproteins 1 (MSP-1) der Merozoitform von *Plasmodium falciparum* von Asn1613 bis Ile1726 kodiert,
und dadurch, dass aufwärts des 5'-terminalen Endes dieser synthetischen Nukleotidsequenz die DNA aufweist:
- die 8 Basenpaare der Leadersequenz des Oberflächenproteins 1 aus Plasmodium vivax, und
- eine Sequenz, die zweiunddreißig Aminosäuren des Oberflächenproteins 1 aus Plasmodium vivax von Met1 bis Asp32 kodiert,
und dadurch, dass abwärts des 3'-terminalen Endes dieser synthetischen Nukleotidsequenz die DNA zwei Stoppkodone TAA aufweist.

11. Rekombinanter Baculovirus-Vektor, der unter Steuerung eines Promotors, der in dem Vektor enthalten ist und geeignet ist, von durch diesen Vektor transfizierbaren Zellen erkannt zu werden, enthält:
- eine DNA gemäß einem der Ansprüche 1 bis 5 und eine Nukleotidsequenz, die ein Peptidsignal aufwärts des 5'-terminalen Endes dieser DNA kodiert, wobei das Peptidsignal geeignet ist, in einem Baculovirus-System als Signal erkannt zu werden, oder
- eine DNA gemäß einem der Ansprüche 6 bis 10.

12. Baculovirus-Vektor gemäß Anspruch 11, **dadurch gekennzeichnet, dass** er aus dem Virus, der beim CNCM unter der Nummer I-1661 hinterlegt ist, und dem Virus, der beim CNCM unter der Nummer I-1662 hinterlegt ist, gewählt ist.

13. Zelle eines Insekts, die mit einem Baculovirus-Vektor gemäß Anspruch 11 oder 12 transfiziert ist.

14. Polypeptidfragment oder Teil eines Polypeptidfragments, wobei das Fragment oder der Teil in der Lage ist, eine Immunantwort zu induzieren, die geeignet ist, eine Parasitämie infolge eines Parasiten *Plasmodium falciparum* zu hemmen, **dadurch gekennzeichnet, dass** das Fragment oder der Teil des Fragments geeignet ist:
- durch einen Baculovirus-Vektor gemäß Anspruch 11 oder 12 exprimiert zu werden, oder
- durch eine Zelle gemäß Anspruch 13 erzeugt zu werden,
und dadurch, dass das Fragment oder der Teil des Fragments
- konformationell instabile Epitope in reduzierendem Medium aufweist, die den Hauptteil der Epitope bilden, die von humanen Antiseren gegen *Plasmodium falciparum* gebildet werden, und
- von humanen Antiseren erkannt wird, die gegen *Plasmodium falciparum* gebildet werden, wenn es im nicht reduzierten Zustand oder in einem nicht irreversibel reduzierten Zustand ist, aber nicht oder nur gering von diesen Antiseren erkannt wird, wenn es im irreversibel reduzierten Zustand ist.

15. Oligomer aus Polypeptidfragmenten oder Teilen von Polypeptidfragmenten gemäß Anspruch 14.

16. Protein mit einem Polypeptidfragment oder Teil eines Polypeptidfragments gemäß Anspruch 14.

17. Zusammensetzung, die als Impfung gegen einen Parasiten *Plasmodium falciparum* zusammengestellt ist, **dadurch gekennzeichnet, dass** sie aufweist:
- ein Polypeptidfragment oder einen Teil eines Polypeptidfragments gemäß Anspruch 14 oder ein Oligomer gemäß Anspruch 15 oder ein Protein gemäß Anspruch 16.

18. Verfahren für die Herstellung eines Polypeptidfragments oder einen Teil eines Polypeptidfragments, wobei das Fragment oder der Teil in der Lage ist, eine Immunantwort zu induzieren, die geeignet ist, eine Parasitämie infolge eines Parasiten *Plasmodium falciparum* zu hemmen, **dadurch gekennzeichnet, dass** es aufweist:
- Einsetzen einer DNA gemäß einem der Ansprüche 1 bis 10 in einen Baculovirus-Vektor, derart, dass der Vektor das durch die DNA kodierte Polypeptidfragment exprimieren kann,
- Transfizieren einer Zelle eines Insekts mit diesem Vektor,
- Gewinnen des Polypeptidfragments oder des Teils des Polypeptidfragments, das/der durch die Zelle erzeugt wurde.
